# EUROPEAN PATENT SPECIFICATION

(11) **EP 3 654 014 B1**
(45) Date of publication and mention of the grant of the patent: **12.06.2024**
(21) Application number: 19208512.4
(22) Date of filing: 12.11.2019
(51) Int. Cl.: G01N 15/14, G01N 15/1433

(54) **BONE MARROW FLUID ANALYSIS METHOD, SAMPLE ANALYZER, AND NON-TRANSITORY STORAGE MEDIUM**
VERFAHREN ZUR ANALYSE VON KNOCHENMARKFLÜSSIGKEIT, PROBENANALYSATOR UND NICHTTRANSITORISCHES SPEICHERMEDIUM
PROCÉDÉ D'ANALYSE DE LIQUIDE CÉPHALORACHIDIEN, ANALYSEUR D'ÉCHANTILLONS ET SUPPORT D'ENREGISTREMENT NON TRANSITOIRE

(30) Priority: 14.11.2018 JP 2018213864
(43) Date of publication of application: 20.05.2020
(73) Proprietor: Juntendo Educational Foundation, Tokyo 113-8421 (JP); SYSMEX CORPORATION, Kobe-shi Hyogo 651-0073 (JP)
(72) Inventor: Ohsaka, Akimichi, Tokyo, 113-8421 (JP); Tabe, Yoko, Tokyo, 113-8421 (JP); Tsuchiya, Koji, Tokyo, 113-8421 (JP); Kimura, Konobu, Hyogo, 651-0073 (JP)
(74) Representative: Hoffmann Eitle

(56) References cited:
- JP-B2- 4 212 827
- US-A1- 2002 086 344
- US-A1- 2003 219 850
- Stuart A. Bentley,, Michael A. Taylor, Donna E. Killian et al.: "Correction of Bone Marrow Nucleated Cell Counts for the Prescence of Fat Particles", American Journal of Clinical Pathology, vol. 104, no. 1 1 July 1995 (1995-07-01), pages 60-64, XP009519609, DOI: 10.1093/ajcp/104.1.60 Retrieved from the Internet: URL:https://academic.oup.com/ajcp/article- abstract/104/1/60/1756093?redirectedFrom=f ulltext [retrieved on 2020-04-01]

## Description

### FIELD OF THE INVENTION

The present invention relates to a bone marrow fluid analysis method, a sample analyzer, and a non-transitory storage medium.

### BACKGROUND

Bone marrow tests are important tests for determining diagnosis and therapeutic effects for blood diseases such as hematopoietic disorder and hematopoietic tumor. Among bone marrow tests, a myelogram test is a useful test that can quickly obtain a result and that is also excellent in cost effectiveness. In the myelogram test, a bone marrow fluid obtained through bone marrow aspiration is smeared on a slide glass and stained, and cells are classified, counted, and morphologically evaluated through microscopy. Making a smear preparation from a bone marrow and observation of cell morphology require specialized knowledge and experience. Thus, myelogram tests are performed by highly trained specialists. For example, in Japan, certificated bone marrow engineers having passed examinations perform myelogram tests, and overseas, specialists of hematopathology such as hematopathologists perform myelogram tests. Japanese Laid-Open Patent Publication No. H4-20298 discloses a pretreatment method and an apparatus for making a preparation for myelogram tests.

One type of information obtained through a myelogram test is bone marrow nucleated cell density. The bone marrow nucleated cell density is obtained by observing cells in a bone marrow fluid by use of a microscope and then obtaining the area ratio between fat cell and nucleated cell (the area of fat cell/the area of nucleated cell) in a smear preparation. In a normal bone marrow fluid, the proportion between nucleated cells and fat cells is substantially constant. However, in bone marrow fluids of patients having blood diseases, abnormal changes are observed in the number of nucleated cells. For example, in a case of a disease such as acute leukemia, the number of nucleated cells in the bone marrow increases, and in a case of a disease such as aplastic anemia, the number of nucleated cells in the bone marrow decreases. At clinical sites, in accordance with the bone marrow nucleated cell density obtained from the area ratio described above, the states of bone marrows are classified into hyperplasia, euplasia, and hypoplasia, to be used for discrimination of blood diseases.

US 2002/0086344 A1 discloses a method for classifying and counting nucleated bone marrow cells comprises the steps of: (1) mixing a sample of bone marrow fluid with an erythrocyte lysing agent to lyse erythrocytes in the sample and render leukocytic cells and erythroid cells in the sample suitable for staining, and staining the sample with a fluorescent dye for producing a difference in intensity of fluorescence between the leukocytic cells and the erythroid cells; (2) introducing the resulting sample to a flow cytometer to detect at least one kind of scattered light and at least one kind of fluorescence; (3) classifying and counting nucleated bone marrow cells, the leukocytic cells and the erythroid cells with use of a difference in the intensity of the fluorescence and the scattered light; (4) calculating the ratio of the nucleated bone marrow cells to the erythroid cells or leukocytic cells from the obtained erythroid cell count or leukocytic cell count and the obtained nucleated bone marrow cell count; and (5) calculating the ratio of the leukocytic cells to the erythroid cells from the erythroid cell count and the leukocytic cell count.

In the paper entitled "Correction of Bone Marrow Nucleated Cell Counts for the Presence of Fat Particles", Stuart A. Bentley et al. analyse the accuracy of corrected TNC counts for fat particles using by a recently-marketed hematological analyzer (Cobas-Helios; Roche Diagnostic Systems, Branchburg, NJ) on 21 marrow samples, using a visual chamber count as the reference method. The correction methods studied were software correction, using the Cobas-Helios differential system, and replacement of the sample plasma with saline. The authors found that uncorrected automated marrow TNC counts (mean, 28.4 × 10'J/T.) were significantly higher than the visual reference counts (mean, 23.1 × ID'/L). The authors conclude that both the corrected automated method, using the Cobas-Helios, and the saline replacement method are acceptable alternatives to the visual chamber count.

US 2003/0219850 A1 discloses an automatic method for analyzing nucleated bone marrow cells comprising partitioning one sample of bone marrow fluid with two samples, one sample being treated with a first lysing agent and a first staining solution and the other sample being treated with a second lysing agent and a second staining solution; and measuring each samples in a flow cytometer using a scattered light and fluoresence which enables to classify and count leukocytes, erythroid cells and lipid particles, as well as mature myeloid cells, lymphoid cells and immature myeloid cells, and to calculate the number of myeloid cells as well as the ratio of myeloid cells and erythroid cells.

### SUMMARY OF THE INVENTION

The scope of the present invention is defined solely by the appended claims, and is not affected to any degree by the statements within this summary.

A bone marrow fluid to be used in a bone marrow test is collected from a patient through bone marrow aspiration. In bone marrow aspiration, local anesthesia is performed on a patient, a paracentesis needle is inserted into the bone marrow, and the bone marrow fluid is aspirated. Bone marrow aspiration often causes pain in the patient, which is a great burden to the patient. Thus, a bone marrow test cannot be performed frequently. Therefore, if a bone marrow test is to be performed, highly accurate information must be obtained. Meanwhile, in a myelogram test, microscopy is performed, and counting and classification are performed manually on the basis of cell morphology. Therefore, the test accuracy of the bone marrow nucleated cell density or the like depends on the technique and experience of the examiner, and could be influenced by subjective factors.

The present inventors have found that an index based on the number of nucleated cells and the number of lipid particles surprisingly has a good correlation with bone marrow nucleated cell density based on microscopy of a bone marrow smear preparation, and have completed the present invention.

An aspect of the present invention provides a computer-implemented method of analyzing bone marrow fluid, including: counting the number of nucleated cells and the number of lipid particles in a bone marrow fluid; and obtaining an index related to bone marrow nucleated cell density, on the basis of the number of nucleated cells and the number of lipid particles.

Here, the "bone marrow fluid" means a bone marrow fluid collected from a subject through bone marrow aspiration or bone marrow biopsy, and a sample containing the bone marrow fluid.

"The number of nucleated cells" means the total of the number of leukocytes and the number of erythroblast cells. Here, leukocytes include myeloblasts, promyelocytes, myelocytes, metamyelocytes, band neutrophils, segmented neutrophils, eosinophils, basophils, lymphocytes, and monocytes, for example. Erythroblast cells are also referred to as nucleated erythrocytes, and include proerythroblasts, basophilic erythroblasts, polychromatic erythroblasts, and orthochromatic erythroblasts, for example.

"The number of lipid particles" means the total number of fat cells and formed components derived from fat cells.

Generally, the bone marrow nucleated cell density is defined as the area ratio (the area of fat cell/the area of nucleated cell) calculated from the area of nucleated cell and the area of fat cell obtained through microscopy of a smear preparation of bone marrow. Here, "index related to bone marrow nucleated cell density" means new information that is related to the bone marrow nucleated cell density, that has been found in the present invention, and that is obtained by use of the number of nucleated cells and the number of lipid particles.

In this aspect, the index related to bone marrow nucleated cell density is a value of a ratio between the number of nucleated cells and the number of lipid particles. In this aspect, the index related to bone marrow nucleated cell density may be a value of a ratio of the number of lipid particles to the number of nucleated cells.

In this aspect, the index related to bone marrow nucleated cell density may be displayed.

In this aspect, the bone marrow fluid analysis method may further include determining a state of a bone marrow on the basis of the number of nucleated cells and the number of lipid particles.

In this aspect, the bone marrow fluid analysis method may further include determining a state of a bone marrow on the basis of the index related to bone marrow nucleated cell density. As the determination of the state of the bone marrow, at least one of hypoplasia, euplasia, or hyperplasia may be determined.

In the above aspect, the state of the bone marrow may be determined by comparing a predetermined threshold with the index related to bone marrow nucleated cell density. In the above aspect, in the determining of the state of the bone marrow, it may be determined that the state of the bone marrow corresponds to hypoplasia when a value of a ratio of the number of lipid particles to the number of nucleated cells is greater than a first threshold, it may be determined that the state of the bone marrow corresponds to euplasia when a value of the ratio of the number of lipid particles to the number of nucleated cells is smaller than the first threshold and is greater than a second threshold, and it may be determined that the state of the bone marrow corresponds to hyperplasia when the value of the ratio of the number of lipid particles to the number of nucleated cells is smaller than the second threshold. The "first threshold" is a threshold for distinguishing hypoplasia from euplasia and hyperplasia. The "second threshold" is a threshold for distinguishing hyperplasia from euplasia and hypoplasia. In the above aspect, information related to the determined state of the bone marrow may be displayed.

In this aspect, the counting of the number of nucleated cells and the number of lipid particles may include measuring the bone marrow fluid through flow cytometry.

In this aspect, the number of nucleated cells and the number of lipid particles in a measurement sample prepared from the bone marrow fluid and a reagent may be counted. In this aspect, the reagent may include at least a hemolytic agent. In this aspect, the reagent may further include a fluorescent dye. Here, the "hemolytic agent" means a substance that can lyse erythrocytes. The "fluorescent dye" means a fluorescent substance that can stain nucleic acid.

In this aspect, the number of lipid particles may be counted on the basis of fluorescence signal information, forward scattered light information, and side scattered light information which have been obtained through flow cytometry. Accordingly, the number of lipid particles can be accurately obtained.

In this aspect, the obtaining of the number of nucleated cells and the number of lipid particles may include: measuring a first measurement sample containing the bone marrow fluid and a first reagent, and counting the number of nucleated cells in the first measurement sample; and measuring a second measurement sample containing the bone marrow fluid and a second reagent different from the first reagent, and counting the number of lipid particles in the second measurement sample.

In this aspect, the first reagent may contain a lysing reagent having a pH of not less than 2.0 and not greater than 4.5, and the second reagent may contain a lysing reagent having a pH of not less than 5.5 and not greater than 7.0.

An aspect of the present invention provides a sample analyzer including: a sample preparation part configured to prepare a measurement sample from a bone marrow fluid; a detector configured to detect particles contained in the measurement sample; and a controller programmed to obtain the number of nucleated cells and the number of lipid particles in the measurement sample on the basis of information obtained by the detector, wherein the controller is programmed to obtain an index related to bone marrow nucleated cell density, on the basis of the number of nucleated cells and the number of lipid particles.

In this aspect, the detector may include: a flow cell configured to allow the measurement sample prepared by the sample preparation part to flow therein; a light source part configured to apply light to the measurement sample flowing in the flow cell; and an optical detector configured to obtain optical information that is obtained when light is applied to the measurement sample.

In this aspect, the number of lipid particles may be obtained on the basis of fluorescence signal information, forward scattered light information, and side scattered light information which have been obtained by the light receiver.

In this aspect, the sample analyzer may further include an output part, and the controller may be programmed to output, to the output part, the index related to bone marrow nucleated cell density. Here, the "output part" includes, for example, a sound output device, a printer, and a display device having a screen on which characters, images and the like can be displayed.

In this aspect, the index related to bone marrow nucleated cell density is a value of a ratio between the number of nucleated cells and the number of lipid particles.

In this aspect, the controller may be programmed to determine a state of a bone marrow on the basis of the number of nucleated cells and the number of lipid particles. In this aspect, the sample analyzer may further include an output part, and the controller may be programmed to output, to the output part, information related to the determined state of the bone marrow.

An aspect of the present invention provides a non-transitory storage medium having stored therein a computer program for analyzing a bone marrow fluid, the computer program configured to cause a computer to perform: obtaining the number of nucleated cells and the number of lipid particles in the bone marrow fluid on the basis of information obtained by a detector configured to detect particles contained in the bone marrow fluid; and obtaining an index of a bone marrow nucleated cell density, on the basis of the number of nucleated cells and the number of lipid particles, wherein the index of bone marrow nucleated cell density is a value of a ratio between the number of nucleated cells and the number of lipid particles.

### BRIEF DESCRIPTION OF THE DRAWINGS

FIG. 1A is a schematic diagram of a scattergram obtained through flow cytometer (FCM) measurement using a nucleated erythrocyte and leukocyte counting reagent;
FIG. 1B is a schematic diagram of a scattergram obtained through FCM measurement using a leukocyte classification reagent;
FIG. 1C is a schematic diagram of a scattergram obtained through FCM measurement using common reagents;
FIG. 2A is a schematic diagram showing a configuration of a sample analyzer of the present embodiment;
FIG. 2B is a schematic diagram showing a configuration of the sample analyzer of the present embodiment;
FIG. 3 is a perspective view showing a configuration of a flow cell;
FIG. 4 is a block diagram showing a configuration of an analysis unit;
FIG. 5 is a flow chart showing a flow of operation performed by the sample analyzer of the present embodiment;
FIG. 6 is a flow chart showing a procedure of a measurement sample preparation process;
FIG. 7 is a flow chart showing a procedure of a measurement data analysis process;
FIG. 8A is a flow chart showing a procedure of a determination process based on bone marrow nucleated cell density;
FIG. 8B is a flow chart showing a procedure of a determination process based on bone marrow nucleated cell density;
FIG. 8C is a flow chart showing a procedure of a determination process based on bone marrow nucleated cell density;
FIG. 9 shows a display example of an analysis result;
FIG. 10A is an example of a scattergram showing distribution of particles in a measurement sample prepared by use of the nucleated erythrocyte and leukocyte counting reagent;
FIG. 10B is a first example of a scattergram showing distribution of particles in a measurement sample prepared by use of the leukocyte classification reagent;
FIG. 10C is a second example of a scattergram showing distribution of particles in a measurement sample prepared by use of the leukocyte classification reagent;
FIG. 11 is a graph showing distribution of the ratio of the number of lipid particles to the number of nucleated cells with respect to subjects classified according to bone marrow nucleated cell density based on microscopy;
FIG. 12A is a ROC curve obtained when whether the bone marrow nucleated cell density corresponded to hypoplasia was determined on the basis of the ratio of the number of lipid particles to the number of nucleated cells; and
FIG. 12B is a ROC curve obtained when whether the bone marrow nucleated cell density corresponded to hyperplasia was determined on the basis of the ratio of the number of lipid particles to the number of nucleated cells.

### DETAILED DESCRIPTION OF THE PREFERRED EMBODIMENTS

### [1. Bone marrow fluid analysis method]

In a bone marrow fluid analysis method of the present embodiment, first, the number of nucleated cells and the number of lipid particles in a measurement sample containing a bone marrow fluid and a reagent are obtained. In the following, a method for obtaining the number of nucleated cells and the number of lipid particles in a bone marrow fluid is described.

### (Bone marrow fluid)

When a bone marrow fluid collected from a subject contains solid foreign matters that could be an obstacle for cell measurement, such as spicule, aggregated blood cells, and the like, the bone marrow fluid may be filtered by use of a mesh, for example. A chelating agent and/or an anticoagulant agent may be added to the bone marrow fluid as necessary. An example of the chelating agent is an EDTA (ethylene diamine tetraacetic acid) salt. An example of the anticoagulant agent is heparin, citric acid, or citrate.

Normally, a bone marrow fluid contains nucleated cells and lipid particles. In a myelogram test, normally, erythroblast cells, leukocytes, plasma cells, reticular cells (macrophage), and megakaryocytes are counted as nucleated cells. Promyelocytes, myelocytes, and metamyelocytes among leukocytes are also comprehensively referred to as granulocytic juvenile cells. Band neutrophils are mature neutrophils, and segmented neutrophils are more mature neutrophils than band neutrophils. Eosinophils include immature eosinophils and mature eosinophils. Basophils include immature basophils and mature basophils.

Leukocytes and erythroblast cells may include tumor cells that emerge from hematopoietic tumors of leukemia, malignant lymphoma, and the like of various types and increase in number. For example, in the case of acute lymphocytic leukemia, lymphoblasts increase in number. Lymphocytes may include atypical lymphocytes. The atypical lymphocytes are lymphocytes activated by antigen priming, and emerge due to viral infection or the like.

In the present embodiment, examples of lipid particles include fat cells, all or part of damaged fat cells, and fat lumps released from damaged fat cells. It should be noted that fat cells have nuclei but are included in lipid particles.

It is known that, in a normal bone marrow, the total of leukocytes and erythroblast cells accounts for 95% to 99% of all the nucleated cells. In the present embodiment, as the nucleated cells, leukocytes and erythroblast cells are measured. That is, in the analysis method of the present embodiment, a measurement sample prepared from a bone marrow fluid is measured, and the total of the number of leukocytes and the number of erythroblast cells is obtained as the number of nucleated cells.

### (Reagent)

A reagent to be used in the analysis method of the present embodiment is not limited in particular as long as the reagent allows measurement of nucleated cells and/or lipid particles. Preferably, the reagent contains at least one type of a hemolytic agent and a fluorescent dye. In a preferable embodiment, the reagent contains a hemolytic agent and a fluorescent dye. In this case, the reagent may be one reagent that contains both of a hemolytic agent and a fluorescent dye. Alternatively, the reagent may be a combination of a lysing reagent containing a hemolytic agent, and a staining reagent containing a fluorescent dye, such reagents being separately prepared. Fluorescent dyes are not stably preserved in aqueous solutions in some cases. Thus, the reagent is preferably composed of two reagents, i.e., a lysing reagent containing a hemolytic agent, and a staining reagent containing a fluorescent dye.

As for the reagent to be used for counting nucleated cells and counting lipid particles, one type of reagent may be used, or two or more types of reagents may be combined to be used. A preferable embodiment employs a combination of a reagent that allows measurement of nucleated cells, and a reagent that allows measurement of lipid particles, such reagents being separately prepared. That is, a lysing reagent and a staining reagent for measuring nucleated cells, and a lysing reagent and a staining reagent for measuring lipid particles are separately used. In the analysis method of the present embodiment, a nucleated erythrocyte and leukocyte counting reagent is used for measurement of nucleated cells. A leukocyte classification reagent is used for measurement of lipid particles. However, this is merely an example, and the reagents to be used for measurement of nucleated cells and measurement of lipid particles are not limited thereto.

### (Nucleated erythrocyte and leukocyte counting reagent)

### 1. Hemolytic agent to be used for measurement of nucleated cells in bone marrow fluid

The nucleated erythrocyte and leukocyte counting reagent enables counting of nucleated erythrocytes and leukocytes (basophils, and leukocytes other than basophils) in a sample. The nucleated erythrocyte and leukocyte counting reagent is composed of two reagents, i.e., a lysing reagent containing a hemolytic agent, and a staining reagent containing a fluorescent dye. As the first reagent described above, the nucleated erythrocyte and leukocyte counting reagent is suitable, but not limited thereto.

The hemolytic agent of the nucleated erythrocyte and leukocyte counting reagent can be selected from a quaternary-ammonium-salt cationic surfactant represented by Formula (1) below, and a pyridinium cationic surfactant represented by Formula (2) below, for example. One type of cationic surfactant may be used, or two or more types of cationic surfactants may be used.

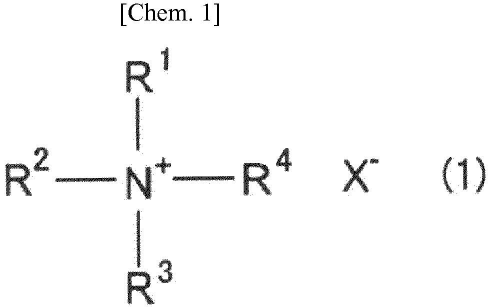

In Formula (1), R¹, R², and R³ are the same or different from each other, and are a hydrogen atom, an alkyl group having 1 to 8 carbon atoms, or an aralkyl group having 6 to 8 carbon atoms; R⁴ is an alkyl group having 8 to 18 carbon atoms, an alkenyl group having 8 to 18 carbon atoms, or an aralkyl group having 6 to 18 carbon atoms; and X⁻ is an anion.

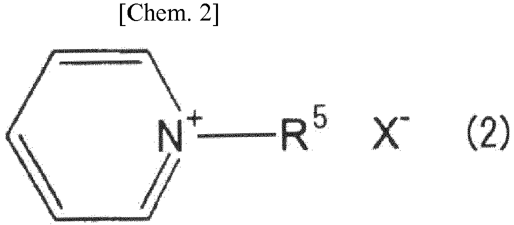

In Formula (2), R⁵ is an alkyl group having 8 to 18 carbon atoms; and X⁻ is an anion.

In Formulas (1) and (2) above, examples of the alkyl group having 1 to 8 carbon atoms include methyl, ethyl, propyl, t-butyl, n-butyl, isopentyl, neopentyl, t-pentyl, isohexyl, heptyl, and octyl. Preferably, the alkyl group having 1 to 8 carbon atoms is an alkyl group having 1 to 3 carbon atoms. Examples of the aralkyl group having 6 to 8 carbon atoms include benzyl and phenethyl.

Examples of the alkyl group having 8 to 18 carbon atoms include octyl, decyl, dodecyl, tetradecyl, hexadecyl, and octadecyl. Preferably, the alkyl group having 8 to 18 carbon atoms is a linear alkyl group having 10 to 14 carbon atoms such as decyl, dodecyl, or tetradecyl. Examples of the alkenyl group having 8 to 18 carbon atoms include octenyl, decenyl, dodecenyl, tetradecenyl, hexadecenyl, and octadecenyl. Examples of the aralkyl group having 6 to 18 carbon atoms include phenylpropylene, phenylbutene, naphthylmethylene, naphthylethylene, naphthylpropylene, biphenylmethylene, and biphenylethylene.

Examples of the anion include a halogen ion (F⁻, Cl⁻, Br⁻, or I⁻), a halogenated boron ion (BF₄⁻, BCl₄⁻, BBr₄⁻, etc.), a phosphorus compound ion, a halogenated oxyacid ion, a fluorosulfate ion, a methylsulfate ion, and a tetraphenylboron compound ion having a halogen or an alkyl group having a halogen as a substituent on an aromatic ring. Among them, Br⁻ or BF₄⁻is preferable.

Examples of the surfactant represented by Formula (1) or (2) include octyltrimethylammonium bromide, octyltrimethylammonium chloride, decyltrimethylammonium bromide, decyltrimethylammonium chloride, dodecyltrimethylammonium bromide, dodecyltrimethylammonium chloride, myristyltrimethylammonium bromide, myristyltrimethylammonium chloride, and dodecylpyridinium chloride.

In the nucleated erythrocyte and leukocyte counting reagent, as the hemolytic agent, it is preferable to use a nonionic surfactant together with the cationic surfactant described above. When the cationic surfactant and the nonionic surfactant are mixed to be used, excessive damage to nucleated erythrocytes and leukocytes by the cationic surfactant can be inhibited. Examples of the nonionic surfactant include polyoxyethylene alkyl ether, polyoxyethylene polyoxypropylene alkyl ether, polyoxyethylene castor oil, polyoxyethylene hydrogenated castor oil, polyoxyethylene sterol, and polyoxyethylene hydrogenated sterol. One type of nonionic surfactant or two or more types of nonionic surfactants may be used.

Examples of the nonionic surfactant include polyoxyethylene (16) oleyl ether, polyoxyethylene (20) cetyl ether, polyoxyethylene (20) polyoxypropylene (8) cetyl ether, polyoxyethylene (30) polyoxypropylene (6) decyltetradecyl ether, polyoxyethylene (20) castor oil, polyoxyethylene (20) hydrogenated castor oil, polyoxyethylene (50) hydrogenated castor oil, and polyoxyethylene (25) phytostanol. Among these, polyoxyethylene (16) oleyl ether and polyoxyethylene (20) polyoxypropylene (8) cetyl ether are particularly preferable.

In the nucleated erythrocyte and leukocyte counting reagent, the concentration of the cationic surfactant in the lysing reagent is normally 300 to 9000 ppm, preferably 400 to 8000 ppm, and more preferably 500 to 7000 ppm. The concentration of the nonionic surfactant in the lysing reagent is normally 500 to 7000 ppm, preferably 800 to 6000 ppm, and more preferably 1000 to 5000 ppm.

The osmotic pressure of the lysing reagent containing the hemolytic agent is normally not greater than 150 mOsm/kg, preferably not greater than 120 mOsm/kg, and more preferably not greater than 100 mOsm/kg. Although the lower limit of the osmotic pressure is not limited in particular, it can be not less than 20 mOsm/kg, preferably not less than 30 mOsm/kg, and more preferably not less than 40 mOsm/kg, for example. The pH of the lysing reagent containing the hemolytic agent is preferably not less than 2.0 and not greater than 4.5, and more preferably not less than 2.5 and not greater than 3.5.

In order to make the osmotic pressure and the pH of the lysing reagent containing the hemolytic agent to be included in the above-described ranges, the lysing reagent preferably contains an electrolyte, a saccharide, a buffer, an aromatic organic acid, or the like, for example. Herein, "aromatic organic acid" means an acid having at least one aromatic ring in the molecule and a salt thereof. As the electrolyte, an inorganic salt is preferable, and examples thereof include sodium chloride and potassium chloride. The saccharide may be any of monosaccharide, disaccharide, polysaccharide, and oligosaccharide, and examples thereof include glucose, lactose, and sucrose. The buffer may be any buffer having a pKa near pH ±2.0 that is set, and examples thereof include malic acid, citric acid, maleic acid, tartaric acid, diglycolic acid, and malonic acid. Examples of the aromatic organic acid include phthalic acid, salicylic acid, benzoic acid, hydroxybenzoic acid, aminobenzoic acid, hippuric acid, p-aminobenzenesulfonic acid, benzenesulfonic acid, and alkali metal salts thereof (for example, a sodium salt and a potassium salt). A suitable concentration of these is about 0.1 to 100 mM, and about 1 to 30 mM is preferable.

### 2. Fluorescent dye to be used for measurement of nucleated cells in bone marrow fluid

An example of the fluorescent dye of the nucleated erythrocyte and leukocyte counting reagent is a compound represented by either one of Formulas (3) and (4) below. One type of fluorescent dye may be used, or two or more types of fluorescent dyes may be used.

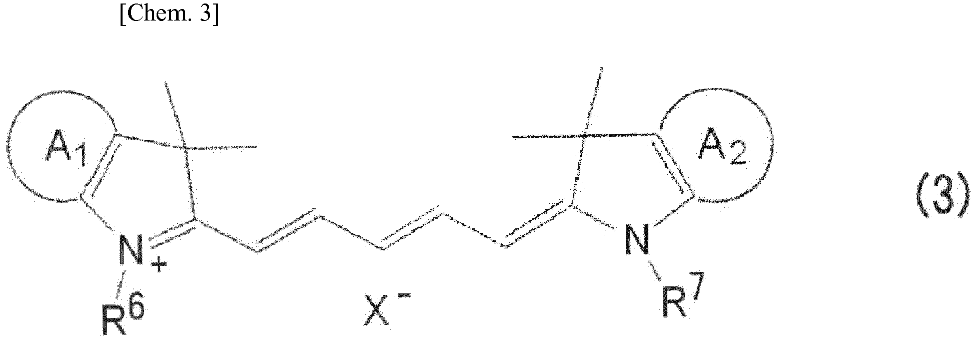

In Formula (3), R⁶ and R⁷ are the same or different from each other and are each an alkyl group; 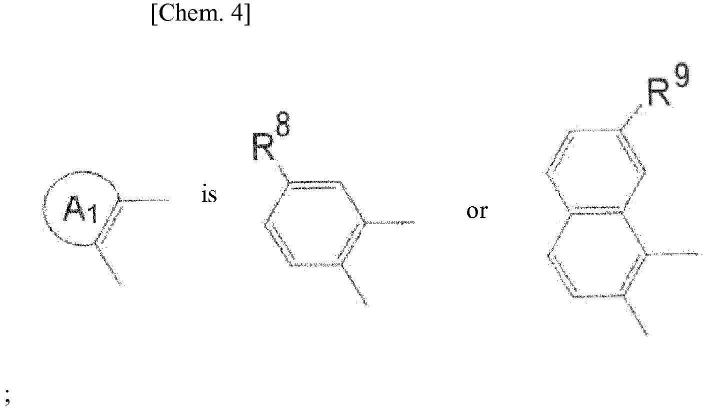 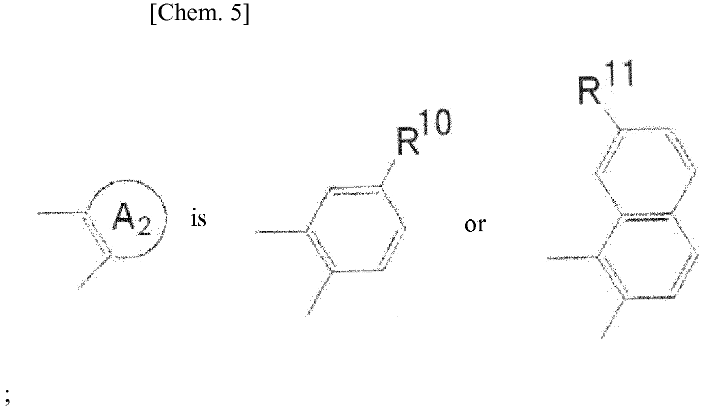 R⁸, R⁹, R¹⁰, and R¹¹ are the same or different from one another, and are each a hydrogen atom or an alkyl group; and X⁻ is an anion.

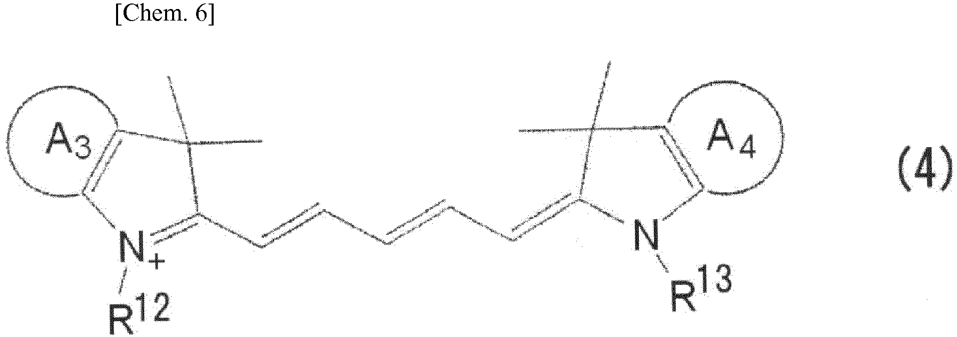

In Formula (4), R¹² and R¹³ are the same or different from each other, and are each an alkyl group optionally containing an acidic group; 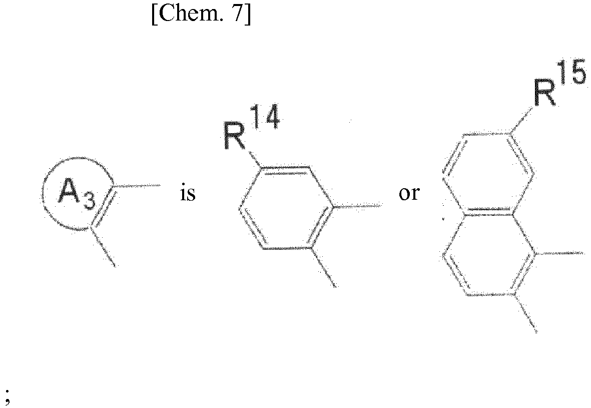 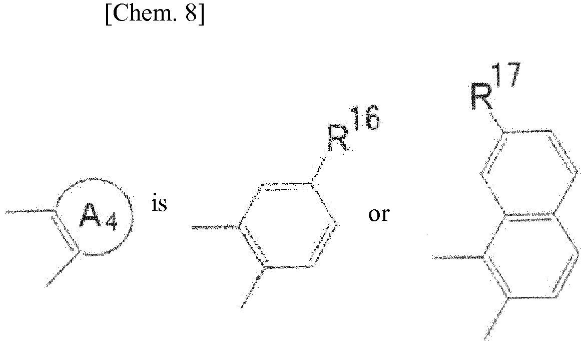 ; R¹⁴, R¹⁵, R¹⁶, and R¹⁷ are the same or different from one another, and are each a hydrogen atom or an acidic group, where an acidic group is present in any one of R¹² to R¹⁷; and an acidic group that may be present in R¹² to R¹⁷ may form a salt, where any one of acidic group(s) that may be present in R¹² to R¹⁷ is a group from which a proton has been released.

The alkyl group in Formula (3) or (4) above may be linear or branched. The number of carbon atoms of the alkyl group is normally 1 to 20, preferably 1 to 10, and more preferably 1 to 6. Examples of the alkyl group include methyl, ethyl, propyl, t-butyl, n-butyl, n-pentyl, and n-hexyl.

Examples of the anion in Formula (3) include halogen ions such as F⁻, Cl⁻, Br⁻, and I⁻, and CF₃SO₃⁻, BF₄⁻, and ClO₄⁻. The acidic group that may be present in Formula (4) includes both a group capable of releasing a proton and a group capable of releasing a proton from which the proton has been released. Examples of the group capable of releasing a proton include a carboxyl group, a sulfonic group, and a phosphate group, and a sulfonic group is particularly preferable. The acidic group may form a salt. Examples of the salt include alkali metal salts such as a sodium salt and a potassium salt. More preferably, the salt is a sodium salt.

Examples of the fluorescent dye represented by Formula (3) or (4) above include NK-529, NK-2670, NK-3750, NK-3383, NK-1840, NK-9001, NK-9003, NK-2929, NK-3375, NK-5056, NK-3266, and NK-3620. These fluorescent dyes are available from Hayashibara Co., Ltd.

The concentration of the fluorescent dye in the reagent can be determined as appropriate in accordance with the type of the fluorescent dye. The concentration of the fluorescent dye is normally not less than 0.01 mg/L and not greater than 100 mg/L, preferably not less than 0.1 mg/L and not greater than 90 mg/L, and more preferably not less than 0.2 mg/L and not greater than 80 mg/L. In a case where the reagent is one reagent containing both a fluorescent dye and a hemolytic agent, the fluorescent dye may be dissolved in the lysing reagent containing the hemolytic agent. In a case where the reagent is a combination of a lysing reagent containing a hemolytic agent and a staining reagent containing a fluorescent dye, the fluorescent dye may be dissolved in an appropriate organic solvent. Such an organic solvent is not limited in particular, as long as the organic solvent allows the fluorescent dye to be dissolved therein. Examples of the organic solvent include an alcohol having 1 to 6 carbon atoms, ethylene glycol, diethylene glycol, polyethylene glycol, and dimethyl sulfoxide (DMSO).

### (Leukocyte classification reagent)

### 1. Hemolytic agent to be used for measurement of lipid particles in bone marrow fluid

The leukocyte classification reagent is a reagent for classifying leukocytes in a sample into three types (granulocyte, lymphocyte, and monocyte), four types (neutrophil and basophil, eosinophil, lymphocyte, and monocyte), five types (neutrophil, basophil, eosinophil, lymphocyte, and monocyte), or six types (neutrophil, basophil, eosinophil, lymphocyte, monocyte, and granulocyte juvenile cell), and counting them. The leukocyte classification reagent also allows counting of lipid particles. The leukocyte classification reagent is composed of two reagents, i.e., a lysing reagent containing a hemolytic agent, and a staining reagent containing a fluorescent dye. As the second reagent described above, the leukocyte classification reagent is suitable, but not limited thereto.

Examples of the hemolytic agent of the leukocyte classification reagent include a combination of a cationic surfactant, a nonionic surfactant, and an aromatic organic acid. In a preferable embodiment, as the lysing reagent containing a hemolytic agent, the following reagent is used: a reagent that contains a cationic surfactant, a nonionic surfactant, and an aromatic organic acid having a concentration of not less than 20 mM and not greater than 50 mM, wherein, when the concentration of the aromatic organic acid is not less than 20 mM and less than 30 mM, the pH of the reagent is not less than 5.5 and not greater than 6.4, and when the concentration of the aromatic organic acid is not less than 30 mM and not greater than 50 mM, the pH of the reagent is not less than 5.5 and not greater than 7.0.

Examples of the aromatic organic acid include phthalic acid, salicylic acid, benzoic acid, hydroxybenzoic acid, aminobenzoic acid, hippuric acid, p-aminobenzenesulfonic acid, benzenesulfonic acid, and alkali metal salts thereof (for example, a sodium salt and a potassium salt). One type of aromatic organic acid may be used, or two or more types of aromatic organic acids may be used. In a case where the lysing reagent includes two or more types of aromatic organic acids, the total of the concentrations thereof only needs to be not less than 20 mM and not greater than 50 mM.

When the concentration of the aromatic organic acid in the lysing reagent is not less than 20 mM and less than 30 mM, the pH of the reagent is preferably not less than 5.5 and not greater than 6.4, and more preferably not less than 5.5 and not greater than 6.2. When the concentration of the aromatic organic acid in the lysing reagent is not less than 30 mM and not greater than 50 mM, and preferably not less than 40 mM and not greater than 50 mM, the pH of the reagent is not less than 5.5 and not greater than 7.0. Further preferably, when the concentration of the aromatic organic acid in the reagent containing a hemolytic agent is not less than 40 mM and not greater than 50 mM, the pH of the reagent is not less than 5.5 and not greater than 6.2.

As the cationic surfactant of the leukocyte classification reagent, a quaternary-ammonium-salt cationic surfactant represented by Formula (5) below, and a pyridinium cationic surfactant represented by Formula (6) below are preferable. One type of cationic surfactant may be used, or two or more types of cationic surfactants may be used.

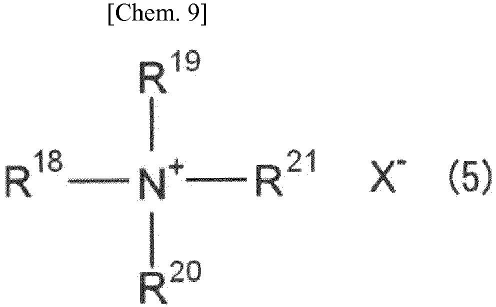

In Formula (5), R¹⁸ is an alkyl group or an alkenyl group having 6 to 18 carbon atoms; R¹⁹ and R²⁰ are the same or different from each other and are each an alkyl group or an alkenyl group having 1 to 4 carbon atoms; R²¹ is an alkyl group or an alkenyl group having 1 to 4 carbon atoms, or a benzyl group; and X⁻ is a halogen ion.

In Formula (5), as R¹⁸, an alkyl group or an alkenyl group having 6, 8, 10, 12, or 14 carbon atoms is preferable, and a linear alkyl group is particularly preferable. Specific examples thereof are an octyl group, a decyl group, and a dodecyl group. As R¹⁹ and R²⁰, a methyl group, an ethyl group, and a propyl group are preferable. As R²¹, a methyl group, an ethyl group, and a propyl group are preferable. Examples of the halogen ion include F⁻, Cl⁻, Br⁻, and I⁻.

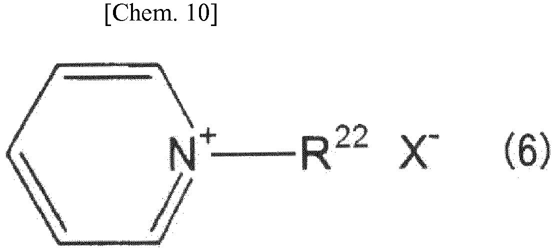

In formula (6), R²² is an alkyl group having 6 to 18 carbon atoms; and X⁻ is a halogen ion. As R²², an alkyl group or an alkenyl group having 6, 8, 10, 12, or 14 carbon atoms is preferable, and a linear alkyl group is particularly preferable. Specific examples thereof include an octyl group, a decyl group, and a dodecyl group. Examples of the halogen ion include F⁻, Cl⁻, Br⁻, and I⁻.

As the nonionic surfactant of the leukocyte classification reagent, a polyoxyethylene-based nonionic surfactant represented by Formula (7) below is preferable.

R²³-R²⁴-(CH₂CH₂O)ₙ-H (7)

In Formula (7), R²³ is an alkyl group, an alkenyl group, or an alkynyl group having 8 to 25 carbon atoms; R²⁴ is an oxygen atom, -COO-, or 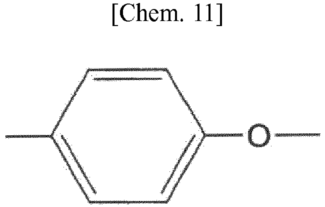 ; and n is 10 to 50.

Examples of the nonionic surfactant above include polyoxyethylene alkyl ether, polyoxyethylene sterol, polyoxyethylene castor oil, polyoxyethylene sorbitan fatty acid ester, polyoxyethylene alkylamine, and polyoxyethylene polyoxypropylene alkyl ether.

In the leukocyte classification reagent, the concentration of the nonionic surfactant in the lysing reagent is 10 to 100000 ppm, preferably 100 to 10000 ppm, and more preferably 1000 to 5000 ppm. The concentration of the cationic surfactant in the reagent is normally 10 to 10000 ppm, and preferably 100 to 1000 ppm.

The lysing reagent of the leukocyte classification reagent may contain a buffer in order to maintain the pH in the ranges described above. Examples of the buffer include citrates, phosphates, and Good's buffers such as HEPES. In a case where an aromatic organic acid having a buffering action is added to the lysing reagent, addition of the buffer is optional. The osmotic pressure of the lysing reagent is not limited in particular, and is preferably not less than 20 mOsm/kg and not greater than 150 mOsm/kg from the viewpoint of efficiently hemolyzing erythrocytes.

### 2. Fluorescent dye to be used for measurement of lipid particles in bone marrow fluid

The fluorescent dye of the leukocyte classification reagent can be selected, for example, from the group consisting of propidium iodide; ethidium bromide; ethidium-acridine heterodimer; ethidium diazide; ethidium homodimer-1; ethidium homodimer-2; ethidium monoazide; trimethylenebis[[3-[[4-[[(3-methylbenzothiazol-3-ium)-2-yl]methylene]-1,4-dihydroquinolin]-1-yl]propyl]dimethylaminium] tetraiodide (TOTO-1); 4-[(3-methylbenzothiazol-2(3H)-yliden)methyl]-1-[3-(trimethylaminio)propyl]quinolinium diiodide (TO-PRO-1); N,N,N',N'-tetramethyl-N,N'-bis[3-[4-[3-[(3-methylbenzothiazol-3-ium)-2-yl]-2-propenylidene]-1,4-dihydroquinolin-1-yl]propyl]-1,3-propanediaminium tetraiodide (TOTO-3); 2-[3-[[1-[3-(trimethylaminio)propyl]-1,4-dihydroquinolin]-4-ylidene]-1-propenyl]-3-methylbenzothiazol-3-ium diiodide (TO-PRO-3); and fluorescent dyes represented by Formula (8) below. One type of fluorescent dye may be used, or two or more types of fluorescent dyes may be used.

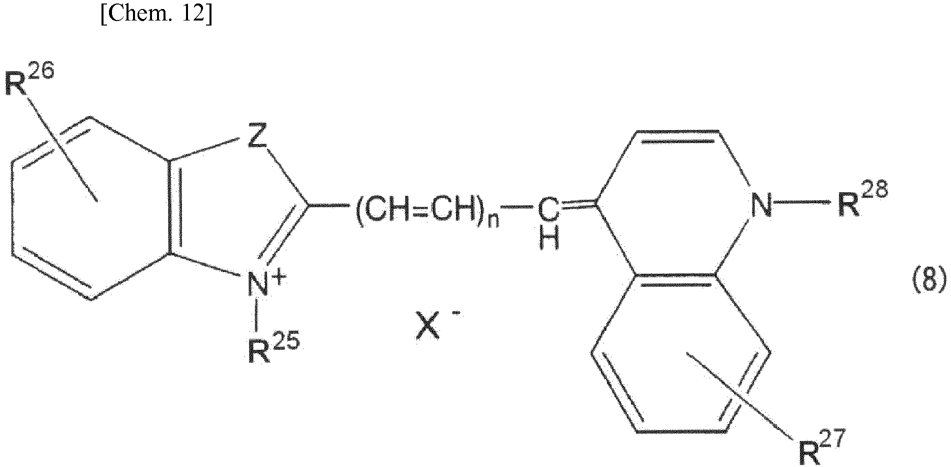

In Formula (8), R²⁵ and R²⁸ are the same or different from each other and are each a hydrogen atom, an alkyl group, an alkyl chain having a hydroxy group, an alkyl chain having an ether group, an alkyl chain having an ester group, or a benzyl group optionally containing a substituent; R²⁶ and R²⁷ are the same or different from each other and are each a hydrogen atom, a hydroxyl group, a halogen, an alkyl group, an alkenyl group, an alkynyl group, an alkoxy group, an alkylsulphonyl group, or a phenyl group; Z is a sulfur atom, an oxygen atom, or a carbon atom having a methyl group; n is 0, 1, 2, or 3; and X⁻ is an anion.

The alkyl group in Formula (8) may be linear or branched. In Formula (8), when either one of R²⁵ and R²⁸ is an alkyl group having 6 to 18 carbon atoms, the other is preferably a hydrogen atom or an alkyl group having less than 6 carbon atoms. Among the alkyl groups having 6 to 18 carbon atoms, an alkyl group having 6, 8, or 10 carbon atoms is preferable. When R²⁵ and/or R²⁸ is a benzyl group optionally containing a substituent, examples of the substituent include an alkyl group having 1 to 20 carbon atoms, an alkenyl group having 2 to 20 carbon atoms, and an alkynyl group having 2 to 20 carbon atoms. Among these, a methyl group or an ethyl group is preferable.

In Formula (8), an example of the alkenyl group of R²⁶ and R²⁷ is an alkenyl group having 2 to 20 carbon atoms. An example of the alkoxy group of R²⁶ and R²⁷ is an alkoxy group having 1 to 20 carbon atoms. Among these, a methoxy group or an ethoxy group is preferable. Examples of the anion in Formula (8) include halogen ions such as F⁻, Cl⁻, Br⁻, and I⁻, and CF₃SO₃⁻, BF₄⁻, and ClO₄⁻.

The concentration and solvent for the fluorescent dye in the leukocyte classification reagent are the same as those described for the nucleated erythrocyte and leukocyte counting reagent.

### (Other reagents)

In the present embodiment, for measurement of nucleated cells and measurement of lipid particles, different lysing reagents and different staining reagents are used, respectively. However, not limited thereto, measurements of nucleated cells and lipid particles may be performed using a lysing reagent and a staining reagent which are commonly used therebetween. For example, as disclosed in Japanese Patent No. 4212827, if a reagent composed of two reagents, i.e., a lysing reagent containing a hemolytic agent and a staining reagent containing a fluorescent dye, is used, measurement of nucleated cells and measurement of lipid particles can be performed using a common lysing reagent and a common staining reagent.

Examples of the hemolytic agent in the common reagent include a surfactant represented by Formula (1), (2), or (7) above, MEGA-8, sucrose monocaprate, deoxy-BIGCHAP, n-octyl-β-D-thioglucoside, n-nonyl- P-D-thiomalto side, n-heptyl-β-D-thioglucoside, n-octyl-β-D-thioglucoside, CHAPS, and CHAPSO. One type of hemolytic agent may be used or two or more types of hemolytic agents may be used.

Examples of the fluorescent dye in the common reagent include NK-2825, NK-1836, NK-1954, Oxazine 750, cryptocyanine, NK-376, NK-382, NK-2711, NK-138, Oxazine 720, LDS 730, LD 700, Nile Blue A, Brilliant Green, Iodide Green, and Malachite Green. One type of fluorescent dye may be used, or two or more types of fluorescent dyes may be used.

### (Preparation and measurement of measurement sample)

In the present embodiment, a measurement sample can be prepared by mixing a bone marrow fluid and a reagent. When the reagent contains a hemolytic agent, at least the bone marrow fluid and the hemolytic agent are mixed together, whereby a measurement sample is prepared. Due to the action of the hemolytic agent in the reagent, nucleated cells in the bone marrow fluid enters a state of being stainable by a fluorescent dye. The state of being stainable by the fluorescent dye means a state where the cell membranes of the cells are damaged to an extent that the fluorescent dye can pass therethrough. In a case where erythrocytes are present in the bone marrow fluid, the erythrocyte can be lysed by the action of the hemolytic agent. Due to the action of the hemolytic agent, similar to erythrocytes, the cell membranes of erythroblast cells are also damaged, but the cell nuclei of the erythroblast cells are maintained. Therefore, the erythroblast cells enter a state of being stainable, and can be classified and counted by a FCM.

In a preferable embodiment, during preparation of a measurement sample, a fluorescent dye is further mixed. In a case where the bone marrow fluid has been treated by the hemolytic agent, the fluorescent dye can enter nucleated cells through the damaged cell membranes, and can stain the nucleic acids in the cell nuclei. In this case, leukocytes are stained intensely and emit intense fluorescence. Erythroblast cells are stained weakly compared with leukocytes and emit weak fluorescence. The mechanism of action that causes a difference in fluorescence intensity between leukocytes and erythroblast cells is not clear. However, it is considered that, probably, since the nuclei (DNA) of erythroblast cells are condensed, intake of the fluorescent dye into the cell nuclei is inhibited. Lipid particles are also stained weakly by the fluorescent dye and emit weak fluorescence. Meanwhile, erythrocytes, cells not having nuclei such as erythrocyte ghosts, and particles are scarcely stained.

In a case where the reagent is one reagent containing a hemolytic agent, or one reagent containing a hemolytic agent and a fluorescent dye, the mixing ratio between the bone marrow fluid and the reagent is normally 1:5-500, and preferably 1:10-100 by volume ratio. In a case where the reagent is a combination of a lysing reagent containing a hemolytic agent and a staining reagent containing a fluorescent dye, the mixing ratio between the bone marrow fluid, the lysing reagent, and the staining reagent is normally 1:5-500:1-10, and preferably 1:10-100:2-5 by volume ratio. Preferably, after the bone marrow fluid and the reagent are mixed together, the mixture is incubated in a predetermined condition. Examples of the predetermined condition include incubation conditions at a temperature of 15 to 50°C, preferably 30 to 45°C, for 5 to 120 seconds, preferably 5 to 30 seconds. Preparation of measurement samples may be performed manually or using an automatic hemocyte analyzer.

In the present embodiment, measurements of nucleated cells and lipid particles in a measurement sample are preferably performed by a FCM. In measurement by the FCM, light is applied to a prepared measurement sample, and optical information is obtained. Specifically, first, a measurement sample is introduced into a flow cell of the FCM, and when each particle in the sample passes through the flow cell, light is applied to the particle. Then, scattered light and fluorescence emitted from the particle are measured, whereby optical information is obtained. Here, the particle means a formed component present in the measurement sample. Examples of the particle include cells, lipid particles, and debris such as remainders of hemolyzed erythrocytes (erythrocyte ghosts). In a case where the reagent contains no fluorescent dye, it is preferable to obtain scattered light information as the optical information. In a case where the reagent contains a fluorescent dye, it is preferable to obtain scattered light information and fluorescence signal information, as the optical information.

Lipid particles might not be sufficiently distinguished from other hemocytes by only two of fluorescence signal information, forward scattered light information, and side scattered light information. Therefore, in the present embodiment, it is preferable to obtain the number of lipid particles on the basis of fluorescence signal information, forward scattered light information, and side scattered light information which are obtained by a flow cytometer.

As the scattered light information, forward scattered light information and side scattered light information are preferable. Examples of the scattered light information include peak of pulse, pulse width, pulse area, and the like of forward scattered light (e.g., light reception angle of around 0 to 20 degrees) and side scattered light (e.g., light reception angle of around 80 to 100 degrees). It is known that forward scattered light reflect the size of a cell, and side scattered light reflects internal information of the nucleus, granules, and the like in a cell. In the present embodiment, it is preferable to obtain forward scattered light intensity and/or side scattered light intensity, as the scattered light information. Examples of the fluorescence signal information include fluorescence intensity, fluorescence pulse width, and fluorescence pulse area. Among them, fluorescence intensity is preferable. The wavelength of excitation light to be applied can be selected as appropriate in accordance with the fluorescent dye.

The FCM is not limited in particular, and a commercially available automatic hemocyte analyzer may be used. Examples of such an apparatus include the XN series of Sysmex Corporation. The light source of the FCM is not limited in particular, and a light source having a wavelength suitable for excitation of the fluorescent dye can be selected as appropriate. As the light source, a blue semiconductor laser, a red semiconductor laser, an argon laser, an He-Ne laser, a mercury arc lamp, or the like is used, for example.

The number of nucleated cells and the number of lipid particles in a measurement sample can be obtained on the basis of the optical information obtained in measurement performed by the FCM. In the present embodiment, it is preferable to create a scattergram having an X-axis and a Y-axis representing two types of information selected from forward scattered light information, side scattered light information, and fluorescence signal information, and analyze the obtained scattergram by an appropriate analysis software, thereby obtaining the number of nucleated cells and the number of lipid particles. Individual particles measured by the FCM are indicated as dots on the scattergram.

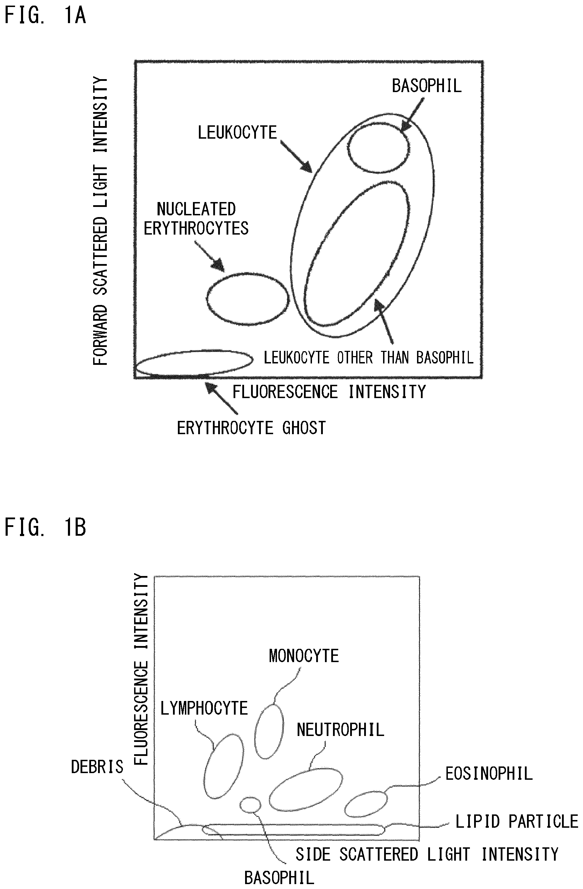

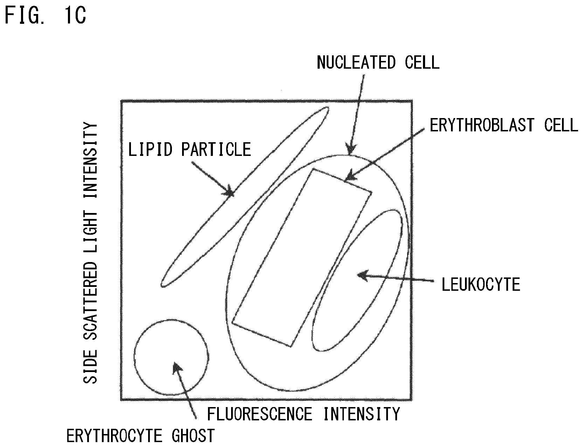

For example, in a case where measurement is performed by use of the nucleated erythrocyte and leukocyte counting reagent, and a scattergram having an X-axis representing fluorescence intensity and a Y-axis representing forward scattered light intensity is created, leukocytes and nucleated erythrocytes are distributed while forming clusters as shown in FIG. 1A. In a case where measurement is performed by use of the leukocyte classification reagent, and a scattergram having an X-axis representing side scattered light intensity and a Y-axis representing fluorescence intensity is created, leukocytes are distributed while forming five types of clusters of lymphocytes, monocytes, neutrophils, eosinophils, and basophils as shown in FIG. 1B. Lipid particles also form a cluster in a region having hardly any fluorescence intensity. In a case where measurement is performed by use of common reagents and a scattergram having an X-axis representing fluorescence intensity and a Y-axis representing side scattered light intensity is created, nucleated cells and lipid particles are distributed while forming clusters as shown in FIG. 1C. The number of cells in each cluster can be obtained by counting the dots in the cluster on the scattergram by means of analysis software installed on the FCM, for example. The scattergrams in FIGS. 1A to 1C are merely examples, and the present disclosure is not limited thereto.

### (Obtainment of index related to bone marrow nucleated cell density)

In an analysis method of the present embodiment, an index related to bone marrow nucleated cell density is obtained on the basis of the number of nucleated cells and the number of lipid particles. In a preferable embodiment, the index related to bone marrow nucleated cell density is information related to bone marrow nucleated cell density that is obtained by use of the number of nucleated cells and the number of lipid particles which are obtained through FCM measurement. The index related to bone marrow nucleated cell density is not limited to the value itself obtained by use of the number of nucleated cells and the number of lipid particles, and may be information that indicates a result, such as hyperplasia, euplasia, or hypoplasia, of determination performed by using the value.

In the present embodiment, the index related to bone marrow nucleated cell density is a value of the ratio between the number of nucleated cells and the number of lipid particles. Examples of the value of the ratio between the number of nucleated cells (NC) and the number of lipid particles (LP) include a ratio (LP/NC) of the number of lipid particles to the number of nucleated cells, a ratio (NC/LP) of the number of nucleated cells to the number of lipid particles, and a value calculated from these ratios. Examples of the value calculated from these ratios include a value obtained by use of an arbitrary coefficient and/or constant in the calculation of the ratio described above. For example, an arbitrary coefficient and/or constant can be used such that the value of LP/NC is substantially on the same order as the value of bone marrow nucleated cell density obtained through microscopy of a smear preparation. Alternatively, the value of the ratio between the number of nucleated cells and the number of lipid particles may be multiplied by 100, so as to express the ratio in percentage. The index related to bone marrow nucleated cell density may be obtained by adding or subtracting an arbitrary value to or from the value of the above ratio as necessary. In a preferable embodiment, the value of the ratio between the number of nucleated cells and the number of lipid particles is preferably LP/NC .

The index related to bone marrow nucleated cell density may be outputted to an output part of the sample analyzer. The output part is preferably implemented as a display, such as a liquid crystal display, a plasma display, or a CRT (Cathode Ray Tube) display mounted in the FCM. The index related to bone marrow nucleated cell density can be used as information that is equivalent to the bone marrow nucleated cell density obtained through microscopy of a smear preparation. Medical professionals such as doctors may use the index related to bone marrow nucleated cell density for discrimination of blood diseases. The index related to bone marrow nucleated cell density is preferably used in combination with other test results and medical observations.

(Determination of bone marrow nucleated cell density) In this technical field, it is determined whether the bone marrow nucleated cell density obtained through microscopy of a smear preparation corresponds to hypoplasia, euplasia, or hyperplasia. As shown in Examples, the index related to bone marrow nucleated cell density obtained by the analysis method of the present embodiment has a good correlation with the bone marrow nucleated cell density obtained through microscopy of a smear preparation. In the analysis method of the present embodiment, the state of the bone marrow may be determined on the basis of the number of nucleated cells and the number of lipid particles. Alternatively, the state of the bone marrow may be determined on the basis of the index related to bone marrow nucleated cell density. For example, at least one determination selected from "whether the state of the bone marrow corresponds to hypoplasia", "whether the state of the bone marrow corresponds to euplasia", and "whether the state of the bone marrow corresponds to hyperplasia" may be performed on the basis of the index related to bone marrow nucleated cell density.

Preferably, the determination above is performed on the basis of a result of comparison between the index related to bone marrow nucleated cell density and a predetermined threshold for the index. For example, when hypoplasia determination is performed by use of LP/NC as the index related to bone marrow nucleated cell density, the value of LP/NC is compared with a first threshold. In the present embodiment, when the value of LP/NC is greater than the first threshold, the state of the bone marrow is determined as corresponding to hypoplasia. When the value of LP/NC is smaller than the first threshold, the state of the bone marrow is determined as corresponding to euplasia or hyperplasia.

In the present embodiment, when the value of LP/NC is the same as the first threshold, the state of the bone marrow may be determined as corresponding to hypoplasia, or alternatively, the state of the bone marrow may be determined as corresponding to euplasia or hyperplasia. That is, when the value of LP/NC is not smaller than the first threshold, the state of the bone marrow may be determined as corresponding to hypoplasia. Alternatively, when the value of LP/NC is not greater than the first threshold, the state of the bone marrow may be determined as corresponding to euplasia or hyperplasia.

For example, when hyperplasia determination is performed by use of LP/NC as the index related to bone marrow nucleated cell density, the value of LP/NC is compared with a second threshold. In the present embodiment, when LP/NC is used as the index related to bone marrow nucleated cell density, the second threshold is a value smaller than the first threshold. When the value of LP/NC is smaller than the second threshold, the state of the bone marrow is determined as corresponding to hyperplasia. When the value of LP/NC is greater than the second threshold, the state of the bone marrow is determined as corresponding to euplasia or hypoplasia.

In the present embodiment, when the value of LP/NC is the same as the second threshold, the state of the bone marrow may be determined as corresponding to hyperplasia, or alternatively, the state of the bone marrow may be determined as corresponding to euplasia or hypoplasia. That is, when the value of LP/NC is not greater than the second threshold, the state of the bone marrow may be determined as corresponding to hyperplasia. Alternatively, when the value of LP/NC is not smaller than the second threshold, the state of the bone marrow may be determined as corresponding to euplasia or hypoplasia.

For example, when euplasia determination is performed by use of LP/NC as the index related to bone marrow nucleated cell density, the value of LP/NC is compared with the first threshold and the second threshold. When the value of LP/NC is smaller than the first threshold and greater than the second threshold, the state of the bone marrow is determined as corresponding to euplasia.

In the present embodiment, when the value of LP/NC is the same as the first threshold, the state of the bone marrow may be determined as corresponding to euplasia. When the value of LP/NC is the same as the second threshold, the state of the bone marrow may be determined as corresponding to euplasia. That is, when the value of LP/NC is not greater than the first threshold and greater than the second threshold, the state of the bone marrow may be determined as corresponding to euplasia. Alternatively, when the value of LP/NC is smaller than the first threshold and not smaller than the second threshold, the state of the bone marrow may be determined as corresponding to euplasia. Alternatively, when the value of LP/NC is not greater than the first threshold and not smaller than the second threshold, the state of the bone marrow may be determined as corresponding to euplasia.

The determination result of the state of the bone marrow may be outputted to the output part described above. Thus, the analysis method of the present embodiment enables provision of information that assists determination of the state of the bone marrow, to medical professionals such as doctors.

The predetermined thresholds are not limited in particular, and can be set as appropriate. For example, the numbers of nucleated cells and the numbers of lipid particles in bone marrow fluids of healthy individuals and patients having various blood diseases are measured, and data is accumulated, whereby the predetermined thresholds may be empirically set. Specifically, the predetermined thresholds may be set in the following manner.

First, bone marrow fluids are collected from subjects including a plurality of healthy individuals and patients having various blood diseases, and a bone marrow nucleated cell density by microscopy of a smear preparation and an index related to bone marrow nucleated cell density by FCM measurement are obtained for each subject. Next, the subjects are classified into a hypoplasia group, a euplasia group, and a hyperplasia group on the basis of the bone marrow nucleated cell densities obtained through microscopy of smear preparations. Then, with respect to the index related to bone marrow nucleated cell density by FCM measurement, values that can most accurately distinguish the groups are obtained, and the values are set as the predetermined thresholds. In setting the thresholds, sensitivity, specificity, positive predictive value, negative predictive value, and the like are preferably taken into consideration.

For example, the first threshold may be set from a range of greater than 0.3 and not greater than 0.75, and preferably not smaller than 0.4 and not greater than 0.5. For example, the second threshold may be set from a range of not smaller than 0.05 and not greater than 0.3, and preferably not smaller than 0.08 and not greater than 0.25.

### [2. Sample analyzer]

In the following, one example of a sample analyzer according to the present embodiment is described with reference to the drawings.

### (Configuration of sample analyzer)

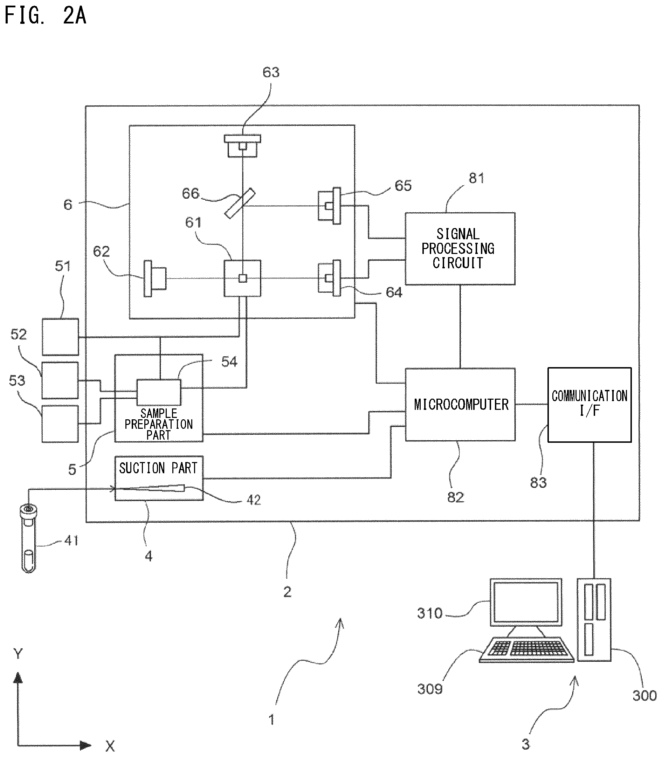

As shown in FIG. 2A, a sample analyzer 1 includes a measurement unit 2 and an analysis unit 3. The measurement unit 2 takes in a bone marrow fluid, prepares a measurement sample from the bone marrow fluid, and performs optical measurement on the measurement sample. The analysis unit 3 processes measurement data obtained through measurement by the measurement unit 2, and outputs a result of analysis of the bone marrow fluid. However, the present embodiment is not limit to this example. For example, the sample analyzer 1 may be an apparatus in which the measurement unit 2 and the analysis unit 3 are integrally formed.

The measurement unit 2 includes a suction part 4, a sample preparation part 5, a detector 6, a signal processing circuit 81, a microcomputer 82, and a communication interface 83. The suction part 4 has a suction tube 42. The suction part 4 suctions a bone marrow fluid contained in a test tube 41, by means of a suction tube 42.

The sample preparation part 5 has a reaction chamber 54, and is connected to reagent containers 51, 52, and 53. The test tube 41 holds a bone marrow fluid. The reagent container 51 holds a diluent. The diluent held in the reagent container 51 is used as a sheath liquid in measurement according to flow cytometry. The reagent container 52 holds a lysing reagent containing a hemolytic agent. The reagent container 53 holds a staining reagent containing a fluorescent dye. The suction part 4 causes the suction tube 42 to move above the reaction chamber 54 and discharge the bone marrow fluid suctioned from the test tube 41, into the reaction chamber 54. The bone marrow fluid, the lysing reagent, and the staining reagent are mixed in the reaction chamber 54, whereby a measurement sample is prepared. In the present embodiment, one reagent containing both a hemolytic agent and a fluorescent dye may be mixed with the bone marrow fluid, to prepare a measurement sample. The measurement sample is subjected to optical measurement according to flow cytometry. In the present embodiment, the number of nucleated cells and the number of lipid particles in the bone marrow fluid are obtained through flow cytometry, but the present disclosure is not limited thereto. For example, an image of a smear preparation of a bone marrow fluid is captured, and image analysis is performed on the captured particle image, whereby the number of nucleated cells and the number of lipid particles may be obtained. Alternatively, the following configuration may be employed. That is, cells are caused to flow in a curved flow path, nucleated cells and lipid particles are caused to flow at different positions so as to be separated according to difference in the magnitude of external force applied on the particles, and separated nucleated cells and lipid particles are counted, whereby the number of nucleated cells and the number of lipid particles are obtained.

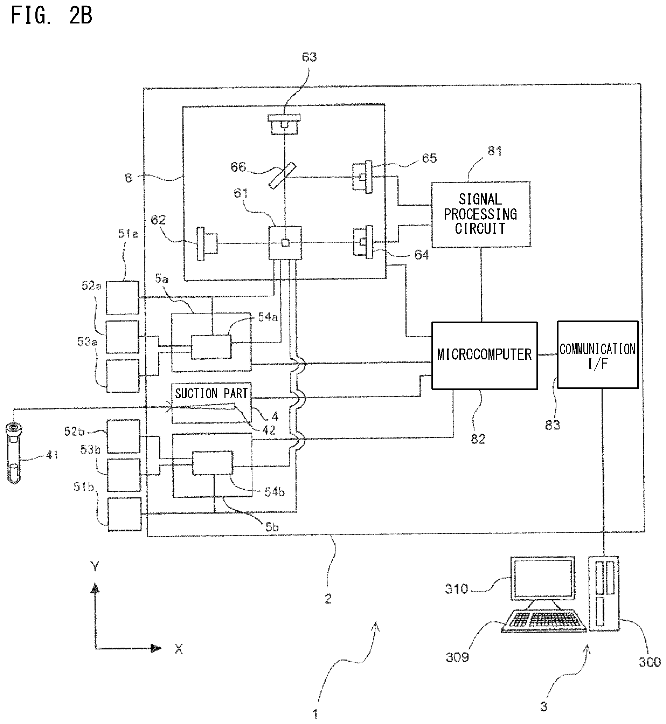

As shown in FIG. 2B, the sample analyzer of the present embodiment may include two or more sample preparation parts. The sample analyzer 1 shown in FIG. 2B is the same as the sample analyzer 1 shown in FIG. 2A except that the measurement unit 2 includes sample preparation parts 5a and 5b. The sample preparation part 5a has a reaction chamber 54a, and is connected to reagent containers 51a, 52a, and 53a. The test tube 41 holds a bone marrow fluid. A sample preparation part 5b has a reaction chamber 54b, and is connected to reagent containers 51b, 52b, and 53b. The reagent containers 51a and 51b each hold a diluent. The reagent containers 52a and 52b each hold a lysing reagent containing a hemolytic agent. In the reagent containers 52a and 52b, the type of the hemolytic agent may be different. The reagent containers 53a and 53b each hold a staining reagent containing a fluorescent dye. In the reagent containers 53a and 53b, the type of the fluorescent dye may be different. For example, the reagent containers 52a and 53a may respectively hold the lysing reagent and the staining reagent of the nucleated erythrocyte and leukocyte counting reagent as the first reagent. The reagent containers 52b and 53b may respectively hold the lysing reagent and the staining reagent of the leukocyte classification reagent as the second reagent. The suction part 4 discharges the bone marrow fluid suctioned from the test tube 41, into each of the reaction chambers 54a and 54b. The bone marrow fluid discharged into the reaction chamber 54a is referred to as a first bone marrow fluid and the bone marrow fluid discharged into the reaction chamber 54b is referred to as a second bone marrow fluid. A first measurement sample is prepared in the reaction chamber 54a, and a second measurement sample is prepared in the reaction chamber 54b.

In the present embodiment, the detector 6 is used in optical measurement of particles according to flow cytometry. The detector 6 includes a flow cell 61, a light source part 62, and light receivers 63, 64, and 65. The flow cell 61 is supplied with the diluent held in the reagent container 51 and a measurement sample prepared by the sample preparation part 5. In the following, a method in which particle detection is performed in the detector 6 according to flow cytometry to obtain the number of nucleated cells and the number of lipid particles is described. However, the present disclosure is not limited thereto. The detector 6 may be provided with an imaging part configured to capture an image of a smear preparation of a bone marrow fluid, and the number of nucleated cells and the number of lipid particles may be obtained on the basis of the particle image captured by the imaging part.

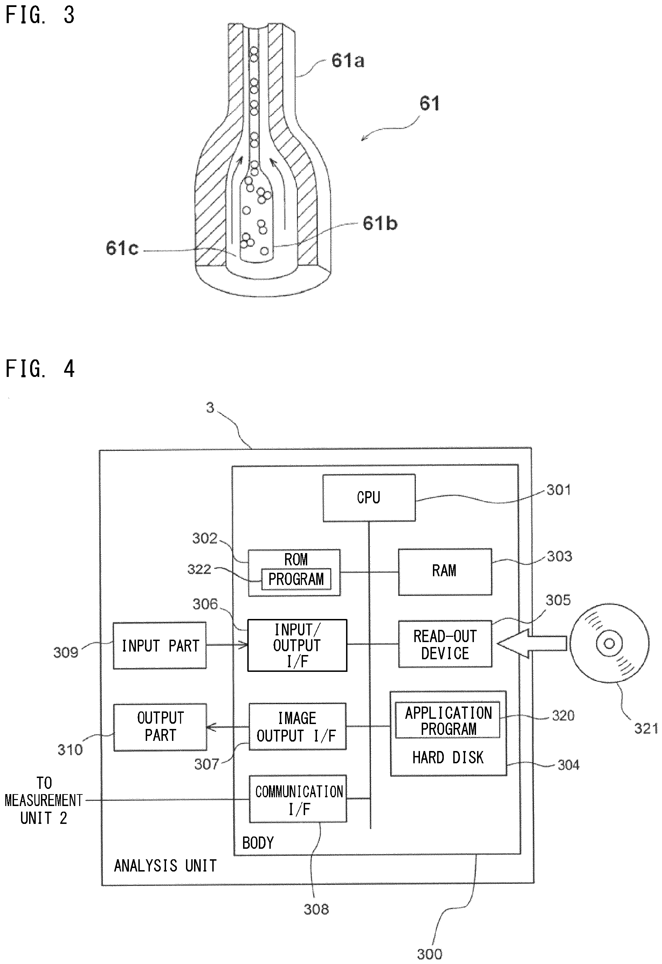

The flow cell 61 is formed in a tube shape with a material such as quartz, glass, or a synthetic resin that has translucency. Inside the flow cell 61, a flow path in which a measurement sample and a sheath liquid flow is provided. With reference to FIG. 3, the flow cell 61 is provided with an orifice 61a whose inner space is narrowed compared with the other portion. The orifice 61a has a double-tube structure near the inlet thereof, and the inner tube portion serves as a sample nozzle 61b. Through the sample nozzle 61b, a measurement sample prepared by the sample preparation part 5 is supplied. The outer space of the sample nozzle 61b serves as a flow path 61c in which the sheath liquid flows. The sheath liquid passes through the flow path 61c and is introduced into the orifice 61a. Thus, the sheath liquid supplied into the flow cell 61 flows so as to envelop the measurement sample discharged from the sample nozzle 61b. Then, the flow of the measurement sample is narrowed by the orifice 61a, whereby particles in the measurement sample enveloped by the sheath liquid passes through the orifice 61a, one by one.

The light source part 62 is a semiconductor laser light source, and applies red laser light having a wavelength of 633 nm, to the orifice 61a of the flow cell 61, for example. The light receivers 63, 64, and 65 each detect light emitted from each individual particle in the measurement sample in the flow cell 61 when light is applied to the flow of the measurement sample. An avalanche photodiode, a photodiode, or a photomultiplier can be employed as the light receiver 63, 64, 65. In the following, the direction connecting the light source part 62 and the flow cell 61 is referred to as "X direction", and a direction orthogonal to the X direction is referred to as "Y direction". A dichroic mirror 66 is disposed to the Y direction side with respect to the flow cell 61. The dichroic mirror 66 allows fluorescence emitted from each particle of the measurement sample, to pass therethrough, and reflects side scattered light emitted from each particle of the measurement sample. The light receiver 63 is disposed to the Y direction side with respect to the flow cell 61, and can detect fluorescence having passed through the dichroic mirror 66. The light receiver 65 can detect side scattered light reflected at the dichroic mirror 66. The light receiver 64 is disposed to the X direction side with respect to the flow cell 61. More specifically, the light receiver 64 is disposed to the side opposite to the light source part 62, with respect to the flow cell 61. The light receiver 64 can detect forward scattered light emitted from each particle of the measurement sample.

The side scattered light is not limited to light that is scattered in a direction at 90° (Y direction) with respect to the optical axis direction (X direction) of the light source part 62. The side scattered light may be light that is scattered in a direction at not less than 80° and not greater than 100° with respect to the X direction, for example. The forward scattered light is not limit to light that is scattered in the optical axis direction (X direction) of the light source part 62. The forward scattered light may be light that is scattered in a direction at not less than -10° and not greater than 10° with respect to the X direction, for example.

In the present embodiment, a light application lens system composed of a plurality of lenses (not shown) may be disposed between the light source part 62 and the flow cell 61. Accordingly, a collimated beam emitted from the semiconductor laser light source can be condensed at a beam spot by the light application lens system.

The light receivers 63, 64, and 65 perform photoelectric conversion on the detected fluorescence, forward scattered light, and side scattered light, respectively, and output analog signals indicating reception light intensities. Hereinafter, the analog signal outputted from the light receiver 63 is referred to as "fluorescence signal", the analog signal outputted from the light receiver 64 is referred to as "forward scattered light signal", and the analog signal outputted from the light receiver 65 is referred to as "side scattered light signal".

The signal processing circuit 81 performs signal processing on the analog signals outputted by the light receivers 63, 64, and 65. The signal processing circuit 81 extracts, as a feature parameter, the peak value of a pulse contained in each of the fluorescence signal, the forward scattered light signal, and the side scattered light signal. Hereinafter, the peak value of the fluorescence signal is referred to as "fluorescence intensity", the peak value of the forward scattered light signal is referred to as "forward scattered light intensity", and the peak value of the side scattered light signal is referred to as "side scattered light intensity".

The microcomputer 82 controls the suction part 4, the sample preparation part 5, the detector 6, the signal processing circuit 81, and the communication interface 83. The communication interface 83 is connected to the analysis unit 3 by a communication cable. The measurement unit 2 performs data communication with the analysis unit 3 via the communication interface 83. The communication interface 83 transmits measurement data containing the feature parameters to the analysis unit 3.

With reference to FIG. 4, a configuration of the analysis unit 3 is described. The analysis unit 3 includes a body 300, an input part 309, and an output part 310. The body 300 includes a CPU (Central Processing Unit) 301, a ROM (Read Only Memory) 302, a RAM (Random Access Memory) 303, a hard disk 304, a read-out device 305, an input/output interface 306, an image output interface 307, and a communication interface 308. In the present embodiment, a display that displays an image is used as the output part 310.

The CPU 301 executes computer programs 322 stored in the ROM 302 and computer programs loaded on the RAM 303. The RAM 303 is used for reading out each computer program stored in the ROM 302 and the hard disk 304. The RAM 303 is also used as a work area for the CPU 301 when executing computer programs. The hard disk 304 has installed therein an application program 320 which is a computer program for analyzing measurement data provided from the measurement unit 2 and outputting an analysis result. The computer program 322 includes a BIOS (Basic Input Output System). The application program 320 includes an OS (Operating System), a bone marrow fluid analysis program, and a bone marrow state determination program. The bone marrow fluid analysis program means a program for obtaining the number of nucleated cells and the number of lipid particles in a bone marrow fluid, and for obtaining an index related to bone marrow nucleated cell density on the basis of the number of nucleated cells and the number of lipid particles. The bone marrow state determination program means a program for determining the state of the bone marrow on the basis of the index related to bone marrow nucleated cell density.

The read-out device 305 is a CD-ROM drive, a DVD-ROM drive, a USB port, an SD card reader, a CF card reader, a memory stick reader, a solid-state drive, a flexible disk drive, or the like, and can read out computer programs and data stored in a portable storage medium 321. The portable storage medium 321 has stored therein the computer program 320 for causing a computer to function as the analysis unit 3. The computer program 320 read out from the portable storage medium 321 is installed into the hard disk 304.

The input part 309 is connected to the input/output interface 306. The output part 310 is connected to the image output interface 307. The communication interface 308 is connected to the communication interface 83 of the measurement unit 2.

### (Operation of sample analyzer)

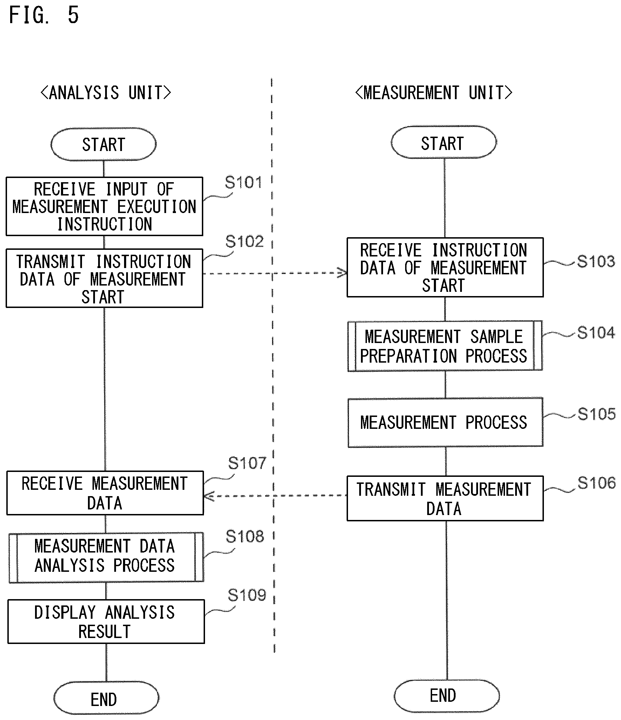

With reference to FIG. 5, operation of the sample analyzer 1 is described. First, the CPU 301 of the analysis unit 3 receives, via the input part 309, a measurement execution instruction from a user (step S101).

The input of the measurement execution instruction is preferably received on the basis of a specimen type selection screen displayed on the output part 310 of the sample analyzer 1. For example, in a case where the sample analyzer 1 can perform measurement of samples of both a blood sample and a body fluid sample including a bone marrow fluid, the sample analyzer 1 can display a specimen type selection screen for designating a specimen type on an input screen for a new analysis order, and allow the user to select a specimen type from blood and body fluid. When the user clicks "measurement start" displayed on the screen after setting a specimen type, a measurement item, and the like, a measurement execution instruction is issued. Selection of the specimen type is not limited to this example. For example, a specific body fluid type such as bone marrow fluid, cerebrospinal fluid, pleural fluid, or ascitic fluid may be designated.

As described above, there are cases where a bone marrow fluid collected from a subject contains solid foreign matters that could be an obstacle for cell measurement, such as spicule, aggregated blood cells, and the like. In addition, a bone marrow fluid and a blood have different concentrations of hemocytes contained therein. Therefore, in a case where the sample analyzer 1 can perform measurement of both a blood and a bone marrow fluid, an additional washing operation as well as a normal washing operation may be performed on the suction part 4 and the flow cell 61 in accordance with the specimen type set at the reception of the input of the measurement execution instruction, in order to suppress influences such as carryover between the blood specimen and the bone marrow fluid specimen. Specifically, in a case where a bone marrow fluid is set as a specimen type, the aforementioned additional washing is performed before the suction part 4 suctions the bone marrow fluid.

When the analysis unit 3 has received a measurement execution instruction, the CPU 301 transmits instruction data that instructs measurement start, to the measurement unit 2 (step S102), and the measurement unit 2 receives the instruction data (step S103). The microcomputer 82 of the measurement unit 2 performs a measurement sample preparation process (step S104) and performs a measurement process (step S105).

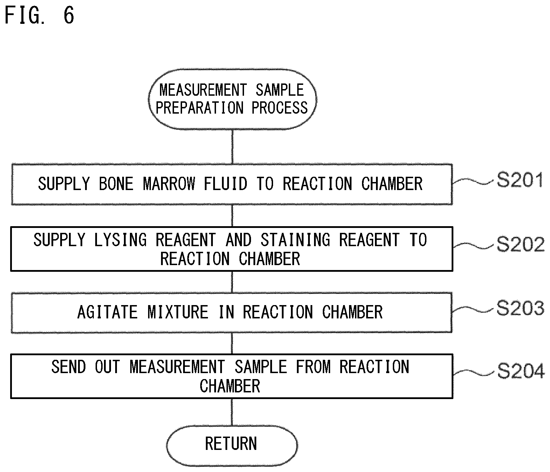

The measurement sample preparation process is described with reference to FIG. 6. The microcomputer 82 controls the suction part 4 to supply a predetermined amount of a bone marrow fluid to the reaction chamber 54 (step S201). Next, the microcomputer 82 controls the sample preparation part 5 to supply a predetermined amount of the first reagent from the reagent container 52 to the reaction chamber 54, and to supply a predetermined amount of the second reagent from the reagent container 53 to the reaction chamber 54 (step S202). The reaction chamber 54 is heated to a predetermined temperature by a heater. In the heated state, the mixture in the reaction chamber 54 is agitated (step S203). Through the operations of step S201 to S203, a measurement sample is prepared in the reaction chamber 54. The microcomputer 82 controls the sample preparation part 5 to send out the measurement sample from the reaction chamber 54 to the detector 6 (step S204). In a case where a measurement unit having a plurality of reaction chambers is used to prepare a plurality of measurement samples such as, for example, a first measurement sample for nucleated cell measurement and a second measurement sample for lipid particle measurement, the above steps are repeated. When the process of step S204 ends, the microcomputer 82 returns the process to the main routine.

With reference to FIG. 5 again, in the measurement process after the measurement sample has been prepared, measurement of the measurement sample by the detector 6 is performed. The sample preparation part 5 supplies the measurement sample together with the sheath liquid to the flow cell 61. When the measurement sample flows in the flow cell 61, particles sequentially pass, one by one, through the orifice 61a of the flow cell 61. The light source part 62 applies light to the measurement sample flowing in the flow cell 61. More specifically, the light source part 62 applies light to each individual particle passing through the orifice 61a of the flow cell 61. Every time light is applied to a particle, fluorescence, forward scattered light, and side scattered light are emitted from the particle.

The fluorescence emitted from the particle is detected by the light receiver 63. The forward scattered light emitted from the particle is detected by the light receiver 64. The side scattered light emitted from the particle is detected by the light receiver 65. The light receivers 63, 64, and 65 output electric signals corresponding to the light reception levels, as a fluorescence signal, a forward scattered light signal, and a side scattered light signal, respectively. The signal processing circuit 81 extracts a fluorescence intensity from the fluorescence signal, extracts a forward scattered light intensity from the forward scattered light signal, and extracts a side scattered light intensity from the side scattered light signal. After the measurement process, the microcomputer 82 transmits measurement data containing feature parameters to the analysis unit 3 (step S106), and ends the process.

The analysis unit 3 receives the measurement data (step S107). Then, the CPU 301 performs a measurement data analysis process, generates an analysis result of the bone marrow fluid, and stores the analysis result into the hard disk 304 (step S108).

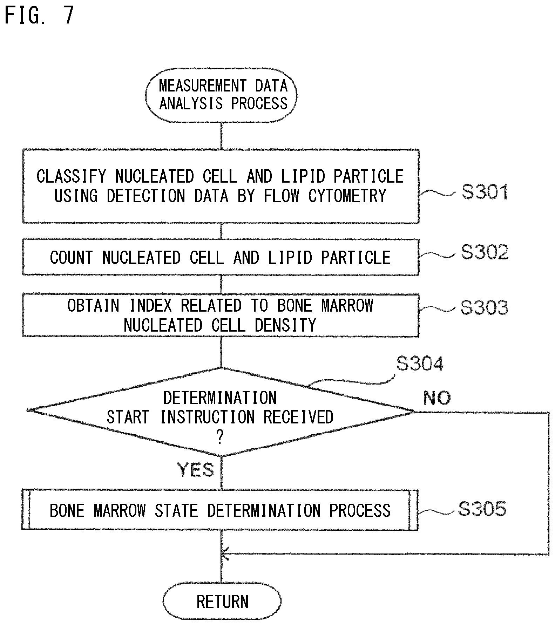

The measurement data analysis process is described with reference to FIG. 7. Upon starting the measurement data analysis process, the CPU 301 of the analysis unit 3 classifies nucleated cells and lipid particles on the basis of the fluorescence intensity, the forward scattered light intensity, and the side scattered light intensity included in the measurement data (step S301). The CPU 301 may create a scattergram by using data of the fluorescence intensity, the forward scattered light intensity, and the side scattered light intensity. In a case where a plurality of measurement samples have been measured, scattergrams may be created for the respective measurement samples on the basis of data of the respective measurement samples.

The process of step S301 is described on the basis of an example case where common reagents are used as the lysing reagent and the staining reagent described above. However, the present disclosure is not limited to this example. In step S301, the CPU 301 combines the particle size distribution of the side scattered light intensity and the particle size distribution of the fluorescence intensity, and specifies a group including leukocytes, a group including erythroblast cells, a group including lipid particles, and a group including erythrocyte ghosts. More specifically, for example, as shown in FIG. 1C, as the group including leukocytes, the CPU 301 specifies a particle group in which both the fluorescence intensity and the side scattered light intensity are at high levels. As the group including erythroblast cells, the CPU 301 specifies a group in which the level of the side scattered light intensity is substantially the same as that of the group including leukocytes, and the level of the fluorescence intensity is lower than that of the group including leukocytes. As the group including lipid particles, the CPU 301 specifies a group in which the level of the side scattered light intensity is substantially the same as that of the group including leukocytes, and the level of the fluorescence intensity is lower than that of the group including erythroblast cells. As the group including erythrocyte ghosts, the CPU 301 specifies a group in which the level of the side scattered light intensity and the level of the fluorescence intensity are lower than those of the group including erythroblast cells.

In step S302, the CPU 301 counts, as the number of nucleated cells, the number of particles in the group including leukocytes and the group including erythroblast cells, which have been classified in step S301, and counts the number of particles in the group including lipid particles. Then, the CPU 301 stores the number of nucleated cells and the number of lipid particles into the hard disk 304. In step S303, the CPU 301 obtains an index related to bone marrow nucleated cell density on the basis of the counted number of nucleated cells and the counted number of lipid particles. In a case where the ratio of the number of lipid particles to the number of nucleated cells is obtained as the index, the CPU 301 divides the number of lipid particles by the number of nucleated cells, thereby obtaining the ratio of the number of lipid particles to the number of nucleated cells. The CPU 301 stores the obtained index related to bone marrow nucleated cell density into the hard disk 304.

In step S304, when the CPU 301 has received via the input part 309 a determination start execution instruction from the user, the CPU 301 determines the state of the bone marrow on the basis of the index related to bone marrow nucleated cell density obtained in step S303. When the CPU 301 has not received the determination start execution instruction from the user, the CPU 301 ends the measurement data analysis process and returns the process to the main routine. With reference to FIG. 5, upon ending the measurement data analysis process described above, the CPU 301 outputs an analysis result to the output part 310 (step S109), and ends the process. In the present embodiment, the determination of the bone marrow state is performed on the basis of the determination start execution instruction from the user. However, the present disclosure is not limited thereto. The determination may be automatically performed without reception of an instruction from the user in particular.

### [3. Computer program]

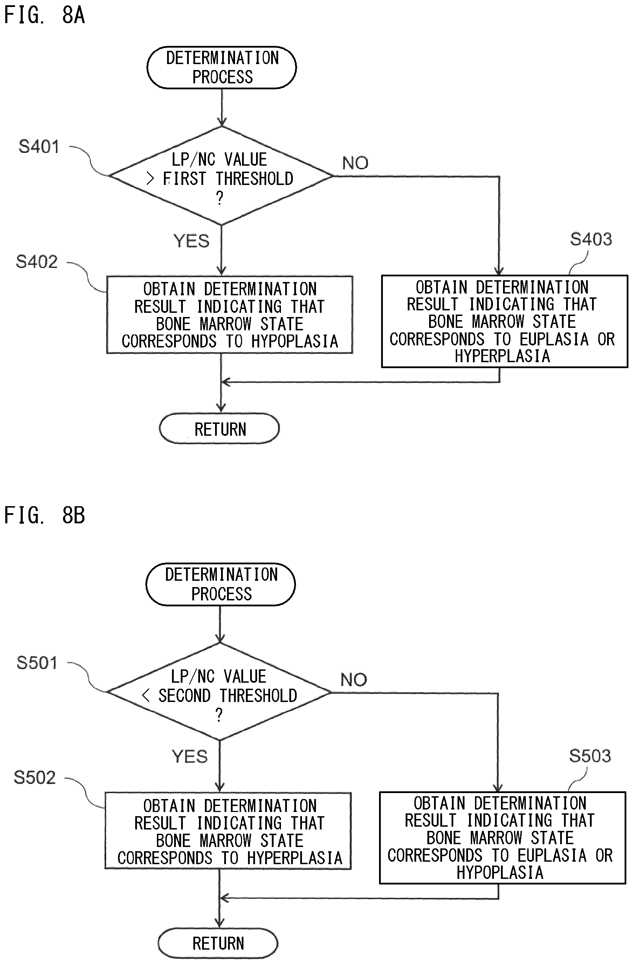

With reference to FIG. 8A, a determination process as to whether the state of the bone marrow corresponds to hypoplasia is described. Here, as an example, a case in which the value of the ratio (LP/NC) of the number of lipid particles to the number of nucleated cells is obtained as the index related to bone marrow nucleated cell density is described. However, the present disclosure is not limited to this example. In step S401, the CPU 301 compares the value of the obtained LP/NC with the first threshold stored in the hard disk 304. When the measurement value is greater than a predetermined threshold, the process advances to step S402. The CPU 301 obtains a determination result indicating that the state of the bone marrow corresponds to hypoplasia, and stores the determination result into the hard disk 304. When the value of the LP/NC is not greater than the first threshold, the process advances to step S403. The CPU 301 obtains a determination result indicating that the state of the bone marrow corresponds to euplasia or hyperplasia, and stores the determination result into the hard disk 304. Then, the CPU 301 ends the determination process and returns the process to the main routine. With reference to FIG. 5, the CPU 301 outputs the determination result to the output part 310 (step S109), and ends the process.

With reference to FIG. 8B, a determination process as to whether the state of the bone marrow corresponds to hyperplasia is described. Here, as an example, a case in which the value of LP/NC is obtained as the index related to bone marrow nucleated cell density is described. However, the present disclosure is not limited to this example. In step S501, the CPU 301 compares the value of the obtained LP/NC with the second threshold stored in the hard disk 304. When the measurement value is smaller than a predetermined threshold, the process advances to step S502. The CPU 301 obtains a determination result indicating that the state of the bone marrow corresponds to hyperplasia, and stores the determination result into the hard disk 304. When the value of the LP/NC is not smaller than the second threshold, the process advances to step S503. The CPU 301 obtains a determination result indicating that the state of the bone marrow corresponds to euplasia or hypoplasia, and stores the determination result into the hard disk 304. Then, the CPU 301 ends the determination process and returns the process to the main routine. With reference to FIG. 5, the CPU 301 outputs the determination result to the output part 310 (step S109), and ends the process.

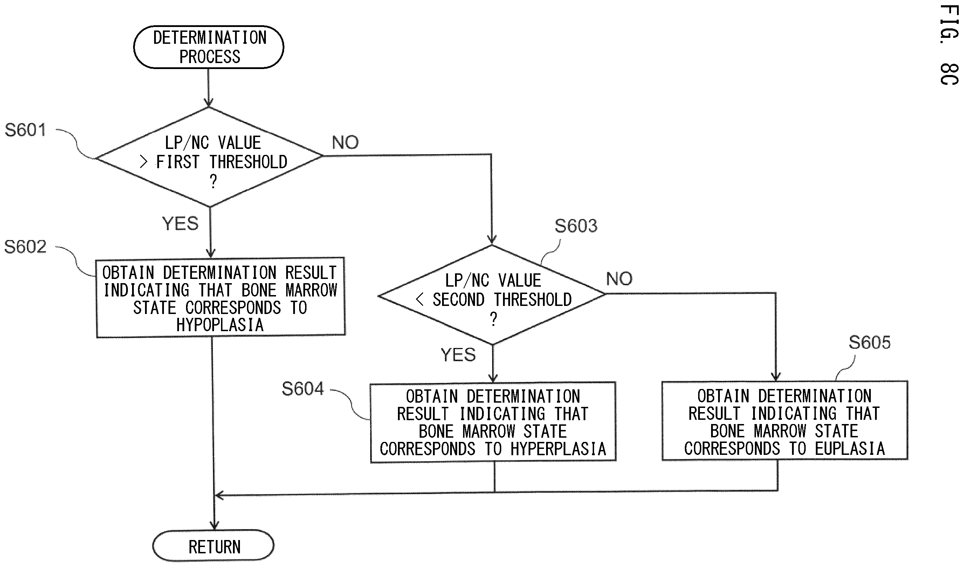

With reference to FIG. 8C, a determination process as to which of hypoplasia, euplasia, or hyperplasia corresponds to the state of the bone marrow is described. Here, as an example, a case in which the value of LP/NC is obtained as the index related to bone marrow nucleated cell density is described. However, the present disclosure is not limited to this example. In step S601, the CPU 301 compares the value of the obtained LP/NC with the first threshold stored in the hard disk 304. When the measurement value is greater than a predetermined threshold, the process advances to step S602. The CPU 301 obtains a determination result indicating that the state of the bone marrow corresponds to hypoplasia, and stores the determination result into the hard disk 304. When the value of the LP/NC is not greater than the first threshold, the process advances to step S603.

In step S603, the CPU 301 compares the value of the obtained LP/NC with the second threshold stored in the hard disk 304. When the measurement value is smaller than a predetermined threshold, the process advances to step S604. The CPU 301 obtains a determination result indicating that the state of the bone marrow corresponds to hyperplasia, and stores the determination result into the hard disk 304. When the value of the LP/NC is not smaller than the second threshold, the process advances to step S605. The CPU 301 obtains a determination result indicating that the state of the bone marrow corresponds to euplasia, and stores the determination result into the hard disk 304. Then, the CPU 301 ends the determination process, and returns the process to the main routine. With reference to FIG. 5, the CPU 301 outputs to the determination result to the output part 310 (step S109), and ends the process.

In the present embodiment, with reference to FIGS. 8A to 8C, "the determination process as to whether the state of the bone marrow corresponds to hypoplasia", "the determination process as to whether the state of the bone marrow corresponds to hyperplasia", and "the determination process as to as to which of hypoplasia, euplasia, or hyperplasia corresponds to the state of the bone marrow" have been separately described. However, as the determination process as to the state of the bone marrow (S305), only a part of these determination processes may be performed, or all the determination processes may be performed.

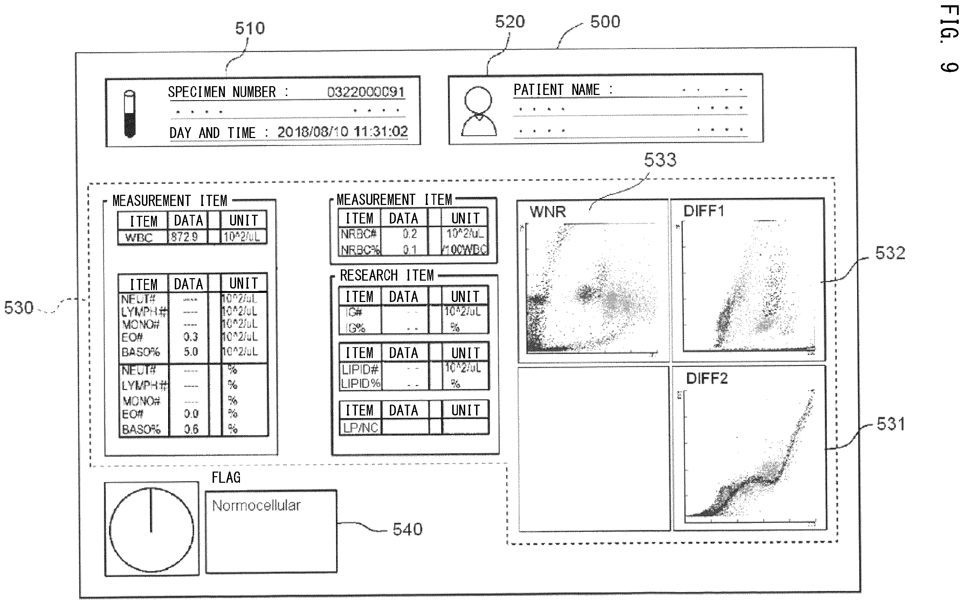

With reference to FIG. 9, an example of an analysis result displayed on the output part 310 is described. However, the present disclosure is not limited to this example. An analysis result screen 500 is displayed on the output part 310. The analysis result screen 500 includes a sample information display region 510, a patient information display region 520, a measurement result display region 530, and a reference information display region 540.

Information of the measured bone marrow fluid is displayed in the sample information display region 510. Information of the subject from whom the bone marrow fluid has been collected is displayed in the patient information display region 520. Measurement values of items obtained through the measurement data analysis process are displayed in the measurement result display region 530. The measurement values displayed in the measurement result display region 530 include the measurement values of the number of leukocytes (WBC), the number of nucleated erythrocytes (NRBC), and the number of lipid particles (LIPID). As the index related to bone marrow nucleated cell density, the value of LP/NC is displayed. The index related to bone marrow nucleated cell density is not limited to the value of LP/NC, and another value may be displayed. The sum of the number of leukocytes (WBC) and the number of nucleated erythrocytes (NRBC) may be displayed as the number of nucleated cells. A scattergram 531 which indicates the distribution of particles in a coordinate space having coordinate axes representing side scattered light intensity and fluorescence intensity, and which has been used in counting the number of lipid particles, and a scattergram 532 which indicates the distribution of particles in a coordinate space having coordinate axes representing side scattered light intensity and forward scattered light intensity, are displayed in the measurement result display region 530. A scattergram 533 which indicates the distribution of particles in a coordinate space having coordinate axes representing fluorescence intensity and forward scattered light intensity and which has been used in counting the number of nucleated cells is displayed in the measurement result display region 530.

When the bone marrow state determination has been performed on the basis of the index related to bone marrow nucleated cell density, a determination result is displayed in the reference information display region 540. In FIG. 9, as a determination result indicating that the state of the bone marrow of the subject corresponds to euplasia, a flag "Normocellular" is displayed on the output part 310. Further, when the state of the bone marrow corresponds to hyperplasia, a flag "Hypercellular" may be displayed on the output part 310, for example. In a case of hypoplasia, a flag "Hypocellular" may be displayed on the output part 310, for example. However, the information indicating the determination result is not limited thereto.

As described above, the sample analyzer and the computer program of the present embodiment can assist diagnosis and discrimination of blood diseases, by providing doctors and the like with the index related to bone marrow nucleated cell density and the determination result based thereon.

In the following, Examples of the present disclosure are described in detail, but the present disclosure is not limited to these Examples.

### [Example]

### Example 1

### (1) Reagent

In Example 1, the first reagent for measuring nucleated cells and the second reagent for measuring lipid particles were used. The first reagent was the nucleated erythrocyte and leukocyte counting reagent composed of two reagents, i.e., a lysing reagent containing a hemolytic agent and a staining reagent containing a fluorescent dye. The second reagent was the leukocyte classification reagent composed of two reagents, i.e., a lysing reagent containing a hemolytic agent and a staining reagent containing a fluorescent dye. The reagents were prepared in the following manner.

### (1.1) First reagent

### Lysing reagent

Dodecyltrimethylammonium chloride (LTAC: Tokyo Chemical Industry Co., Ltd.), polyoxyethylene (20) polyoxypropylene (8) cetyl ether (PBC-44: Nikko Chemicals Co., Ltd.), potassium hydrogen phthalate (FUJIFILM Wako Pure Chemical Corporation), and DL-malic acid and EDTA-2K (Chubu Chelest Co Ltd.) were mixed together to realize the following composition. Purified water was used as a solvent. The pH of the reagent was adjusted to 3.0 by use of sodium hydroxide.

### [Composition of lysing reagent]

2000 ppm LTAC
1000 ppm PBC-44
2 mM potassium hydrogen phthalate
10 mM DL-malic acid
0.2 g/L EDTA-2K
0.0324 g/LNaOH

### Staining reagent

NK-3383 (Hayashibara Co., Ltd.) as a fluorescent dye was dissolved in ethylene glycol. The concentration of NK-3383 in the staining reagent was 51.1 mg/L.

### (1.2) Second reagent

### Lysing reagent

LTAC (Tokyo Chemical Industry Co., Ltd.), polyoxyethylene (30) cetyl ether (BC30TX: Nikko Chemicals Co., Ltd.), potassium hydrogen phthalate (FUJIFILM Wako Pure Chemical Corporation), and EDTA-2K (Chubu Chelest Co Ltd.) were mixed together to realize the following composition. Purified water was used as a solvent. The pH of the reagent was adjusted to 6.0 by use of sodium hydroxide.

### [Composition of lysing reagent]

685 ppm LTAC
1750 ppm BC30TX
40 mM potassium hydrogen phthalate
0.2 g/L EDTA-2K
1.431 g/LNaOH

### Staining reagent

STROMATOLYSER-4DS (Sysmex Corporation) was used as the staining reagent of the second reagent.

### (2) Measurement

### (2.1) Preparation of measurement sample

Bone marrow fluids collected from 18 subjects (EDTA-2K-added bone marrow fluids) were used. Apart of the bone marrow fluid of each subject was taken and stained according to a usual method, to make a smear preparation. The remaining bone marrow fluid was filtered with a nylon mesh having a pore size of 40 µm, to remove spicules. The bone marrow fluid was measured by an automatic hemocyte analyzer XN-2000 (Sysmex Corporation) equipped with a FCM. First, the bone marrow fluid was separated into a first bone marrow fluid and a second bone marrow fluid. The lysing reagent (50 µL) and the staining reagent (1 µL) of the first reagent were added to the first bone marrow fluid (1 µL), and the mixture was incubated at 40°C for 20 seconds, to prepare a first measurement sample. The lysing reagent (50 µL) and the staining reagent (1 µL) of the second reagent were added to the second bone marrow fluid (1 µL), and the mixture was incubated at 40°C for 20 seconds, to prepare a second measurement sample.

### (2.2) Measurement of measurement sample

Light was applied to the first measurement sample, and optical signals emitted from each particle in the sample were obtained. In addition, light was applied to the second measurement sample, and optical signals emitted from each particle in the sample were obtained. In Example 1, fluorescence intensity, side scattered light intensity, and forward scattered light intensity were obtained as optical information. On the basis of the optical signals obtained through the measurement of each measurement sample, scattergrams were created. Distribution of bone marrow fluids and preparation and measurement of measurement samples were automatically performed by XN-2000.

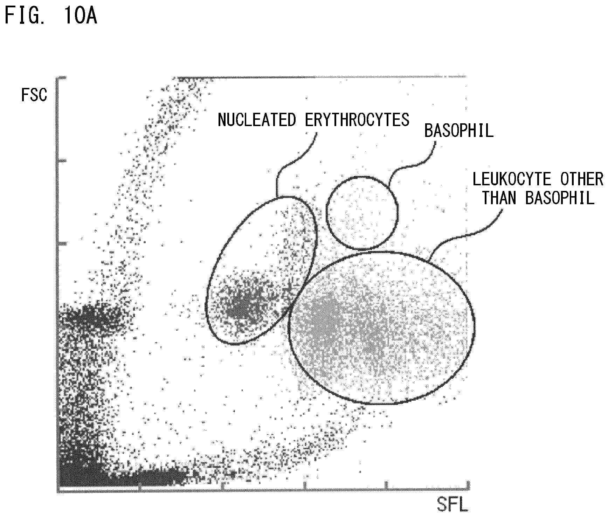

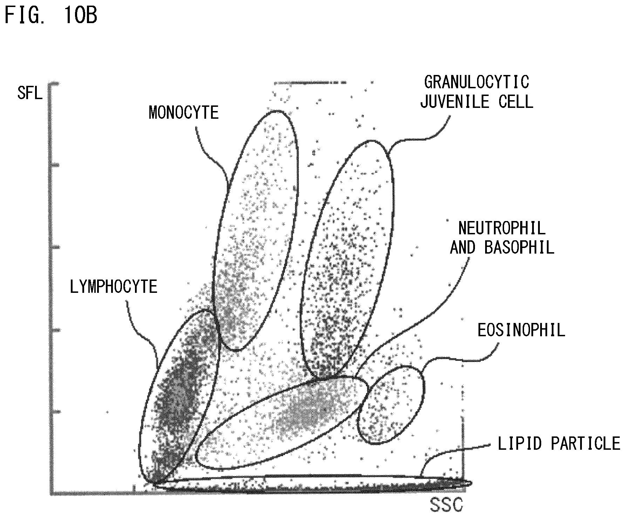

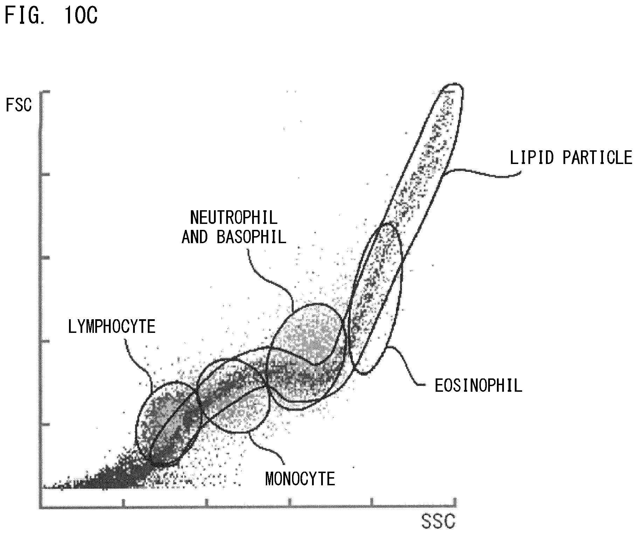

Examples of the created scattergrams are shown in FIGS. 10A, 10B, and 10C. FIG. 10A is a scattergram of measurement using the first reagent (the nucleated erythrocyte and leukocyte counting reagent). FIGS. 10B and 10C are each a scattergram of measurement using the second reagent (the leukocyte classification reagent). In FIG. 10A, the X-axis represents fluorescence intensity and the Y-axis represents forward scattered light intensity. In FIG. 10B, the X-axis represents side scattered light intensity, and the Y-axis represents fluorescence intensity. In FIG. 10C, the X-axis represents side scattered light intensity, and the Y-axis represents forward scattered light intensity. In FIG. 10A, as shown in regions surrounded by ellipses, a cluster of nucleated erythrocytes, a cluster of basophils, and a cluster of leukocytes other than basophils emerged as nucleated cell clusters. In FIG. 10B, as shown in regions surrounded by ellipses, leukocytes were classified into five types of clusters: a cluster of lymphocytes; a cluster of monocytes; a cluster of neutrophils and basophils; a cluster of eosinophils; and a cluster of granulocytic juvenile cells. A cluster of lipid particles emerged so as to extend along the X-axis, as a cluster in a region having hardly any fluorescence intensity. In FIG. 10C, leukocytes were classified into four types of clusters: a cluster of lymphocytes; a cluster of monocytes, a cluster of neutrophils and basophils; and a cluster of eosinophils. A cluster of lipid particles emerged in a region having a meandering shape in the scattergram. In Example 1, the number of nucleated cells was obtained on the basis of the fluorescence intensity and the forward scattered light intensity which were obtained through measurement using the nucleated erythrocyte and leukocyte counting reagent. The number of lipid particles was obtained on the basis of the fluorescence intensity, the side scattered light intensity, and the forward scattered light intensity which were obtained through measurement using the leukocyte classification reagent.

### (3) Analysis and result

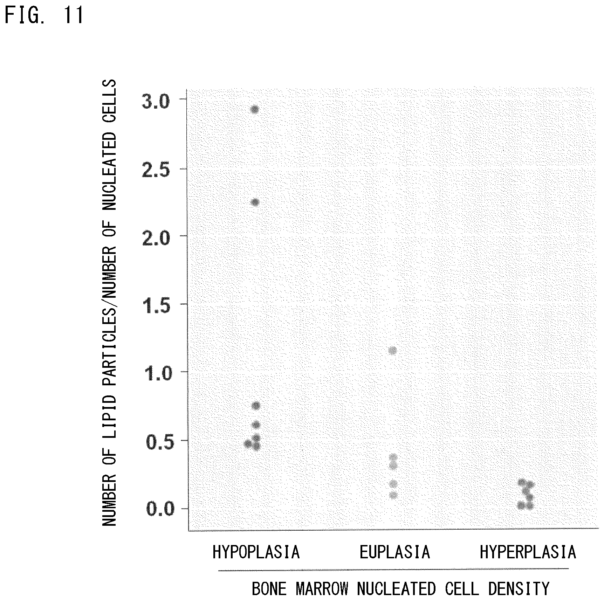

Each smear preparation was observed by use of a microscope, a bone marrow nucleated cell density of each subject was obtained, and the state of the bone marrow was determined. The result revealed that 7 subjects were hypoplasia cases, 5 subjects were euplasia cases, and 6 subjects were hyperplasia cases. With respect to the bone marrow fluid of each subject, the ratio (number of lipid particles/number of nucleated cells) of the number of lipid particles to the number of nucleated cells was calculated from the number of nucleated cells and the number of lipid particles obtained by the XN-2000. With respect to the subjects classified according to the bone marrow nucleated cell densities based on microscopy, distribution of the ratio of the number of lipid particles to the number of nucleated cells was studied. FIG. 11 shows the result.

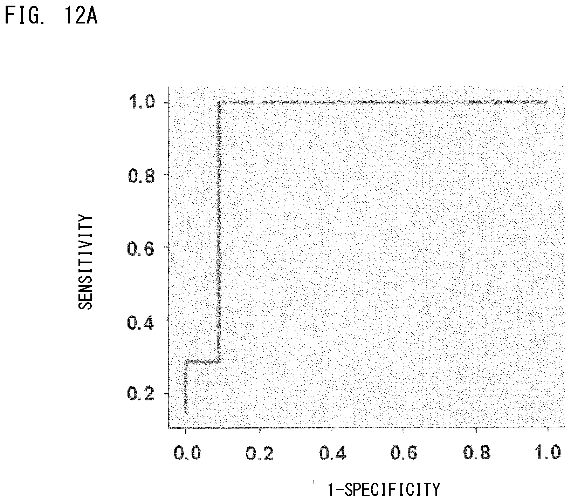

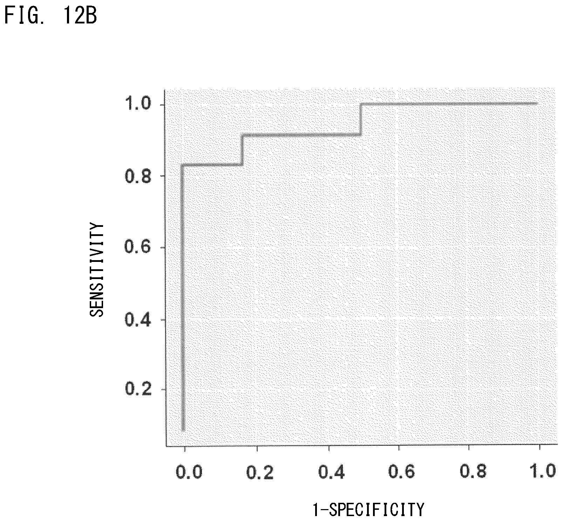

With respect to the value of the ratio of the number of lipid particles to the number of nucleated cells, an optimum cutoff value for discriminating subjects of hypoplasia from the other subjects (euplasia and hyperplasia) was obtained through ROC analysis. The result revealed that, when the cutoff value for the value of the ratio was 0.45, the sensitivity was 100.0% and the specificity was 85.7%. An optimum cutoff value for discriminating subjects of hyperplasia from the other subjects (euplasia and hypoplasia) was also obtained. The result revealed that, when the cutoff value for the ratio of the number of lipid particles to the number of nucleated cells was 0.17, the sensitivity was 94.1% and the specificity was 90.9%. FIGS. 12A and 12B show the obtained ROC curves.

### (4) Discussion

As seen from FIG. 11, the correlation between the bone marrow nucleated cell density based on microscopy and the ratio of the number of lipid particles to the number of nucleated cells obtained by the automatic hemocyte analyzer was good. The above result shows that, by measuring a bone marrow fluid by use of a FCM, it is possible to obtain the ratio of the number of lipid particles to the number of nucleated cells as the information related to the bone marrow nucleated cell density. The result also shows that the state of the bone marrow can be accurately determined on the basis of the ratio of the number of lipid particles to the number of nucleated cells.

## Claims

1. A computer-implemented method of analyzing bone marrow fluid, comprising:
counting (S302) the number of nucleated cells and the number of lipid particles in a bone marrow fluid on the basis of information obtained by a detector (6) configured to detect particles contained in the bone marrow fluid; and
obtaining (S303) an index of bone marrow nucleated cell density, on the basis of the number of nucleated cells and the number of lipid particles,
**characterised in that**
the index of bone marrow nucleated cell density is a value of a ratio between the number of nucleated cells and the number of lipid particles.

2. The method of claim 1, wherein
the index of bone marrow nucleated cell density is a value of a ratio of the number of lipid particles to the number of nucleated cells.

3. The method of any one of claim 1 or 2, further comprising
displaying the index of bone marrow nucleated cell density.

4. The method of any one of claims 1 to 3, further comprising
determining a state of a bone marrow on the basis of the index of bone marrow nucleated cell density.

5. The method of claim 4, wherein
the determining of the state of the bone marrow includes determining at least one of hypoplasia, euplasia, or hyperplasia.

6. The method of claim 4 or 5, wherein
the determining of the state of the bone marrow includes determining the state of the bone marrow by comparing a predetermined threshold with the index of bone marrow nucleated cell density.

7. The method of any one of claims 4 to 6, wherein
the index of bone marrow nucleated cell density is a value of a ratio of the number of lipid particles to the number of nucleated cells, and
the determining of the state of the bone marrow includes:
determining that the state of the bone marrow corresponds to hypoplasia when the index of bone marrow nucleated cell density is greater than a first threshold;
determining that the state of the bone marrow corresponds to euplasia when the index of bone marrow nucleated cell density is smaller than the first threshold and is greater than a second threshold; and
determining that the state of the bone marrow corresponds to hyperplasia when the index of bone marrow nucleated cell density is smaller than the second threshold.

8. The method of any one of claims 4 to 7, further comprising
displaying information related to the determined state of the bone marrow.

9. The method of any one of claims 1 to 8, wherein
the counting of the number of nucleated cells and the number of lipid particles includes measuring the bone marrow fluid through flow cytometry.

10. The method of claim 9, wherein
the counting of the number of lipid particles includes counting the number of lipid particles on the basis of fluorescence signal information, forward scattered light information, and side scattered light information which have been obtained through the flow cytometry.

11. The method of any one of claims 1 to 10, wherein
the counting of the number of nucleated cells and the number of lipid particles includes:
measuring a first measurement sample containing the bone marrow fluid and a first reagent, and counting the number of nucleated cells in the first measurement sample; and
measuring a second measurement sample containing the bone marrow fluid and a second reagent different from the first reagent, and counting the number of lipid particles in the second measurement sample.

12. The method of claim 11, wherein
the first reagent includes a lysing reagent having a pH of not less than 2.0 and not greater than 4.5, and
the second reagent includes a lysing reagent having a pH of not less than 5.5 and not greater than 7.0.

13. A sample analyzer (1) comprising:
a sample preparation part (5) configured to prepare a measurement sample from a bone marrow fluid;
a detector (6) configured to detect particles contained in the measurement sample; and
a controller (301) programmed to count the number of nucleated cells and the number of lipid particles in the measurement sample on the basis of information obtained by the detector (6), and obtain an index of bone marrow nucleated cell density, on the basis of the number of nucleated cells and the number of lipid particles,
**characterised in that**
the index of bone marrow nucleated cell density is a value of a ratio between the number of nucleated cells and the number of lipid particles.

14. A non-transitory storage medium (321) having stored therein a computer program (320) for analyzing a bone marrow fluid, the computer program (320) configured to cause a computer (3) to perform:
counting (S302) the number of nucleated cells and the number of lipid particles in the bone marrow fluid on the basis of information obtained by a detector (6) configured to detect particles contained in the bone marrow fluid; and
obtaining (S303) an index of bone marrow nucleated cell density, on the basis of the number of nucleated cells and the number of lipid particles,
**characterised in that**
the index of bone marrow nucleated cell density is a value of a ratio between the number of nucleated cells and the number of lipid particles.

## Patentansprüche

1. Computerimplementiertes Verfahren zur Analyse von Knochenmarkflüssigkeit, umfassend:
Zählen (S302) der Anzahl von kernhaltigen Zellen und der Anzahl von Lipidpartikeln in einer Knochenmarkflüssigkeit auf der Grundlage von Informationen, die von einem Detektor (6) erhalten werden, der so konfiguriert ist, dass er in der Knochenmarkflüssigkeit enthaltene Partikel erkennt; und
Gewinnen (S303) eines Indexes der Dichte der kernhaltigen Zellen des Knochenmarks auf der Grundlage der Anzahl der kernhaltigen Zellen und der Anzahl der Lipidpartikel,
**dadurch gekennzeichnet, dass**
der Index der Dichte der kernhaltigen Zellen des Knochenmarks ein Wert ist, der das Verhältnis zwischen der Anzahl der kernhaltigen Zellen und der Anzahl der Lipidpartikel angibt.

2. Verfahren nach Anspruch 1, wobei
der Index der Dichte der kernhaltigen Zellen des Knochenmarks ein Wert ist, der das Verhältnis der Anzahl der Lipidpartikel zur Anzahl der kernhaltigen Zellen angibt.

3. Verfahren nach Anspruch 1 oder 2, weiter umfassend
Anzeigen des Index der Dichte der kernhaltigen Zellen des Knochenmarks.

4. Verfahren nach einem der Ansprüche 1 bis 3, weiter umfassend
Bestimmen eines Zustands eines Knochenmarks auf der Grundlage des Index der Dichte der kernhaltigen Zellen des Knochenmarks.

5. Verfahren nach Anspruch 4, wobei
das Bestimmen des Zustands des Knochenmarks das Bestimmen mindestens einer von Hypoplasie, Euplasie oder Hyperplasie einschließt.

6. Verfahren nach Anspruch 4 oder 5, wobei
das Bestimmen des Zustands des Knochenmarks das Bestimmen des Zustands des Knochenmarks durch Vergleichen eines vorbestimmten Schwellenwerts mit dem Index der Dichte der kernhaltigen Zellen des Knochenmarks einschließt.

7. Verfahren nach einem der Ansprüche 4 bis 6, wobei
der Index der Dichte der kernhaltigen Zellen des Knochenmarks ein Wert ist, der das Verhältnis der Anzahl der Lipidpartikel zur Anzahl der kernhaltigen Zellen angibt, und
das Bestimmen des Zustands des Knochenmarks Folgendes einschließt:
Bestimmen, dass der Zustand des Knochenmarks einer Hypoplasie entspricht, wenn der Index der Dichte der kernhaltigen Zellen des Knochenmarks größer als ein erster Schwellenwert ist;
Bestimmen, dass der Zustand des Knochenmarks einer Euplasie entspricht, wenn der Index der Dichte der kernhaltigen Zellen des Knochenmarks kleiner als der erste Schwellenwert und größer als ein zweiter Schwellenwert ist; und
Bestimmen, dass der Zustand des Knochenmarks einer Hyperplasie entspricht, wenn der Index der Dichte der kernhaltigen Zellen des Knochenmarks kleiner als der zweite Schwellenwert ist.

8. Verfahren nach einem der Ansprüche 4 bis 7, weiter umfassend
Anzeigen von Informationen über den bestimmten Zustand des Knochenmarks.

9. Verfahren nach einem der Ansprüche 1 bis 8, wobei
das Zählen der Anzahl der kernhaltigen Zellen und der Anzahl der Lipidpartikel das Messen der Knochenmarkflüssigkeit mittels Durchflusszytometrie einschließt.

10. Verfahren nach Anspruch 9, wobei
das Zählen der Anzahl von Lipidpartikeln das Zählen der Anzahl von Lipidpartikeln auf der Grundlage von Fluoreszenzsignalinformationen, Vorwärtsstreulichtinformationen und Seitenstreulichtinformationen, die durch die Durchflusszytometrie erhalten wurden, einschließt.

11. Verfahren nach einem der Ansprüche 1 bis 10, wobei
das Zählen der Anzahl der kernhaltigen Zellen und der Anzahl der Lipidpartikel Folgendes einschließt:
Messen einer ersten Messprobe, die die Knochenmarkflüssigkeit und ein erstes Reagenz enthält, und Zählen der Anzahl der kernhaltigen Zellen in der ersten Messprobe; und
Messen einer zweiten Messprobe, die die Knochenmarkflüssigkeit und ein zweites Reagenz enthält, das sich von dem ersten Reagenz unterscheidet, und Zählen der Anzahl der Lipidpartikel in der zweiten Messprobe.

12. Verfahren nach Anspruch 11, wobei
das erste Reagenz ein lysierendes Reagenz mit einem pH-Wert von nicht weniger als 2,0 und nicht mehr als 4,5 einschließt, und
das zweite Reagenz ein lysierendes Reagenz mit einem pH-Wert von nicht weniger als 5,5 und nicht mehr als 7,0 einschließt.

13. Probenanalysator (1), umfassend:
einen Probenvorbereitungsteil (5), der so konfiguriert ist, dass er eine Messprobe aus einer Knochenmarkflüssigkeit vorbereitet;
einen Detektor (6), der so konfiguriert ist, dass er in der Messprobe enthaltene Partikel erkennt; und
eine Steuereinheit (301), die so konfiguriert ist, dass sie die Anzahl der kernhaltigen Zellen und die Anzahl der Lipidpartikel in der Messprobe auf der Grundlage der vom Detektor (6) erhaltenen Informationen zählt und einen Index der Dichte der kernhaltigen Zellen des Knochenmarks auf der Grundlage der Anzahl der kernhaltigen Zellen und der Anzahl der Lipidpartikel erhält,
**dadurch gekennzeichnet, dass**
der Index der Dichte der kernhaltigen Zellen des Knochenmarks ein Wert ist, der das Verhältnis zwischen der Anzahl der kernhaltigen Zellen und der Anzahl der Lipidpartikel angibt.

14. Nichttransitorisches Speichermedium (321), in dem ein Computerprogramm (320) zum Analysieren einer Knochenmarkflüssigkeit gespeichert ist, wobei das Computerprogramm (320) so konfiguriert ist, dass es einen Computer (3) veranlasst, Folgendes durchzuführen:
Zählen (S302) der Anzahl von kernhaltigen Zellen und der Anzahl von Lipidpartikeln in der Knochenmarkflüssigkeit auf der Grundlage von Informationen, die von einem Detektor (6) erhalten werden, der so konfiguriert ist, dass er in der Knochenmarkflüssigkeit enthaltene Partikel erkennt; und
Gewinnen (S303) eines Indexes der Dichte der kernhaltigen Zellen des Knochenmarks auf der Grundlage der Anzahl der kernhaltigen Zellen und der Anzahl der Lipidpartikel,
**dadurch gekennzeichnet, dass**
der Index der Dichte der kernhaltigen Zellen des Knochenmarks ein Wert ist, der das Verhältnis zwischen der Anzahl der kernhaltigen Zellen und der Anzahl der Lipidpartikel angibt.

## Revendications

1. Procédé mis en oeuvre par ordinateur permettant d'analyser un liquide de moelle osseuse, comprenant :
le comptage (S302) du nombre de cellules nucléées et du nombre de particules lipidiques dans un liquide de moelle osseuse sur la base des informations obtenues par un détecteur (6) configuré pour détecter des particules contenues dans le liquide de moelle osseuse ; et
l'obtention (S303) d'un indice de densité de cellules nucléées de la moelle osseuse, sur la base du nombre de cellules nucléées et du nombre de particules lipidiques,
**caractérisé en ce que**
l'indice de densité des cellules nucléées de la moelle osseuse est une valeur du rapport entre le nombre de cellules nucléées et le nombre de particules lipidiques.

2. Procédé selon la revendication 1, dans lequel
l'indice de densité des cellules nucléées de la moelle osseuse est une valeur du rapport entre le nombre de particules lipidiques et le nombre de cellules nucléées.

3. Procédé selon l'une quelconque des revendications 1 ou 2, comprenant en outre
l'affichage de l'indice de densité des cellules nucléées de la moelle osseuse.

4. Procédé selon l'une quelconque des revendications 1 à 3, comprenant en outre
la détermination d'un état d'une moelle osseuse sur la base de l'indice de densité des cellules nucléées de la moelle osseuse.

5. Procédé selon la revendication 4, dans lequel
la détermination de l'état de la moelle osseuse inclut la détermination d'au moins l'une parmi hypoplasie, euplasie ou hyperplasie.

6. Procédé selon la revendication 4 ou la revendication 5, dans lequel
la détermination de l'état de la moelle osseuse inclut la détermination de l'état de la moelle osseuse par la comparaison d'un seuil prédéterminé avec l'indice de densité des cellules nucléées de la moelle osseuse.

7. Procédé selon l'une quelconque des revendications 4 à 6, dans lequel
l'indice de densité des cellules nucléées de la moelle osseuse est une valeur du rapport entre le nombre de particules lipidiques et le nombre de cellules nucléées, et
la détermination de l'état de la moelle osseuse inclut :
la détermination que l'état de la moelle osseuse correspond à une hypoplasie lorsque l'indice de densité des cellules nucléées de la moelle osseuse est supérieur à un premier seuil ;
la détermination que l'état de la moelle osseuse correspond à une euplasie lorsque l'indice de densité des cellules nucléées de la moelle osseuse est inférieur au premier seuil et supérieur à un second seuil ; et
la détermination que l'état de la moelle osseuse correspond à une hyperplasie lorsque l'indice de densité des cellules nucléées de la moelle osseuse est inférieur au second seuil.

8. Procédé selon l'une quelconque des revendications 4 à 7, comprenant en outre
l'affichage d'informations relatives à l'état déterminé de la moelle osseuse.

9. Procédé selon l'une quelconque des revendications 1 à 8, dans lequel
le comptage du nombre de cellules nucléées et du nombre de particules lipidiques inclut la mesure du liquide de moelle osseuse par cytométrie de flux.

10. Procédé selon la revendication 9, dans lequel
le comptage du nombre de particules lipidiques inclut le comptage du nombre de particules lipidiques sur la base d'informations de signal de fluorescence, d'informations de lumière diffusée vers l'avant, et d'informations de lumière diffusée vers le côté qui ont été obtenues par la cytométrie de flux.

11. Procédé selon l'une quelconque des revendications 1 à 10, dans lequel
le comptage du nombre de cellules nucléées et du nombre de particules lipidiques inclut :
la mesure d'un premier échantillon de mesure contenant le liquide de moelle osseuse et un premier réactif, et le comptage du nombre de cellules nucléées dans le premier échantillon de mesure ; et
la mesure d'un second échantillon de mesure contenant le liquide de moelle osseuse et un second réactif différent du premier réactif et le comptage du nombre de particules lipidiques dans le second échantillon de mesure.

12. Procédé selon la revendication 11, dans lequel
le premier réactif inclut un réactif de lyse dont le pH est compris entre 2,0 et 4,5, et
le second réactif comprend un réactif de lyse dont le pH est compris entre 5,5 et 7,0.

13. Analyseur d'échantillons (1) comprenant :
une partie (5) de préparation d'échantillon configurée pour préparer un échantillon de mesure à partir d'un liquide de moelle osseuse ;
un détecteur (6) configuré pour détecter les particules contenues dans l'échantillon de mesure ; et
un dispositif de commande (301) programmé pour compter le nombre de cellules nucléées et le nombre de particules lipidiques dans l'échantillon de mesure sur la base d'informations obtenues par le détecteur (6), et pour obtenir un indice de la densité des cellules nucléées de la moelle osseuse, sur la base du nombre de cellules nucléées et du nombre de particules lipidiques,
**caractérisé en ce que**
l'indice de densité des cellules nucléées de la moelle osseuse est une valeur du rapport entre le nombre de cellules nucléées et le nombre de particules lipidiques.

14. Support de stockage non transitoire (321) sur lequel est stocké un programme informatique (320) pour l'analyse d'un liquide de moelle osseuse, le programme informatique (320) étant configuré pour permettre à un ordinateur (3) de mettre en oeuvre :
le comptage (S302) du nombre de cellules nucléées et du nombre de particules lipidiques dans le liquide de moelle osseuse sur la base d'informations obtenues par un détecteur (6) configuré pour détecter les particules contenues dans le liquide de moelle osseuse ; et
l'obtention (S303) d'un indice de densité des cellules nucléées de la moelle osseuse, sur la base du nombre de cellules nucléées et du nombre de particules lipidiques,
**caractérisé en ce que**
l'indice de densité des cellules nucléées de la moelle osseuse est une valeur du rapport entre le nombre de cellules nucléées et le nombre de particules lipidiques.
